(19) **Europäisches Patentamt / European Patent Office / Office européen des brevets**

(11) **EP 4 652 964 A1**

(12) **EUROPEAN PATENT APPLICATION**
published in accordance with Art. 153(4) EPC

(43) Date of publication:
26.11.2025 Bulletin 2025/48

(21) Application number: 24744310.4

(22) Date of filing: 17.01.2024

(51) International Patent Classification (IPC):
$A61C\ 19/04$ (2006.01)   $G06T\ 17/00$ (2006.01)
$A61C\ 7/00$ (2006.01)

(52) Cooperative Patent Classification (CPC):
A61C 7/002; A61C 7/00; A61C 19/04; G06T 17/00;
G16H 20/30; G16H 40/63; G16H 50/20;
G16H 50/50; G16H 50/70

(86) International application number:
PCT/CN2024/072805

(87) International publication number:
WO 2024/153136 (25.07.2024 Gazette 2024/30)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 17.01.2023  CN 202310081632
17.01.2023  CN 202310081504

(71) Applicant: Shanghai Ea Medical Instruments
Company Limited
Shanghai 200433 (CN)

(72) Inventors:
• WANG, Mingzheng
Shanghai 200433 (CN)
• FENG, Yang
Shanghai 200433 (CN)

(74) Representative: Ipsilon
Le Centralis
63, avenue du Général Leclerc
92340 Bourg-la-Reine (FR)

(54) **TOOTH DEVIATION ESTIMATION METHOD AND SYSTEM**

(57) A tooth deviation estimation method and system. The method comprises: acquiring a first three-dimensional digital model and a second three-dimensional digital model, the second three-dimensional digital model being a three-dimensional digital model obtained by performing tooth collection on a patient, and the first three-dimensional digital model being one of a series of successive three-dimensional digital models in an orthodontic treatment scheme using a tray-shaped tooth aligner; obtaining a first reference point set and a second reference point set according to the first three-dimensional digital model and the second three-dimensional digital model; on the basis of the first reference point set and the second reference point set, registering at least one tooth to be estimated in the first three-dimensional digital model with said at least one tooth in the second three-dimensional digital model; and according to the registration result of said at least one tooth in the first three-dimensional digital model and said at least one tooth in the second three-dimensional digital model, obtaining estimation information of said at least one tooth.

```
┌─────────────────────────────────────────────────┐
│ Obtaining first and second three-dimensional      │─110
│ digital models                                     │
└─────────────────────────────────────────────────┘
                      ↓
┌─────────────────────────────────────────────────┐
│ Obtaining a first reference point set and a        │─120
│ second reference point set according to the first  │
│ and the second three-dimensional digital models    │
└─────────────────────────────────────────────────┘
                      ↓
┌─────────────────────────────────────────────────┐
│ Based on the first reference point set and the     │─130
│ second reference point set, performing registration│
│ between at least one tooth to be estimated of the  │
│ first three-dimensional digital model and at least │
│ one tooth to be estimated of the second            │
│ three-dimensional digital model                    │
└─────────────────────────────────────────────────┘
```

FIG. 1

## Description

### Technical Field

The present application generally relates to a method and system for estimating tooth deviation.

### Background

With the continuous development of computer science, dental professionals are increasingly relying on computer technology to improve the efficiency of dental treatment.

The three-dimensional digital model of dentition is one of the most commonly used data in dental treatment. Typically, the three-dimensional digital model of the dentition can be obtained by intraoral scanning, or by scanning a physical model (e.g., a plaster model) or an impression of the dento-jaw.

In the process of orthodontic treatment, the actual treatment effect of a patient may be inconsistent with the treatment plan. Therefore, dental professionals need to compare the patient's current tooth arrangement with the corresponding tooth arrangement in the treatment plan during or after the orthodontic treatment to evaluate the actual treatment effect.

Existing methods mainly rely on manual recognition and judgment, and are mainly based on two-dimensional data, which have drawbacks such as low efficiency, significant influence from subjective factors, and low accuracy.

### Summary

The present application provides a method for estimating tooth deviation, including: obtaining first and second three-dimensional digital models; where the first and the second three-dimensional digital model represent different three-dimensional digital models of the same patient; the second three-dimensional digital model is a three-dimensional digital model obtained by scanning teeth of the patient, and the first three-dimensional digital model is one of a series of successive three-dimensional digital models in an orthodontic treatment plan using a shell-style orthodontic appliance; obtaining a first reference point set and a second reference point set based on the first and the second three-dimensional digital models; where the first reference point set includes a reference point of at least one tooth to be estimated of the first three-dimensional digital model, the second reference point set includes a reference point of at least one tooth of the second three-dimensional digital model corresponding to the reference point in the first reference point set; based on the first and the second reference point sets, preforming registration between the at least one tooth to be estimated of the first three-dimensional digital model and at least one tooth to be estimated of the second three-dimensional digital model; based on a result of the registration between the at least one tooth to be estimated of the first second three-dimensional digital model and at least one tooth to be estimated of the second three-dimensional digital model, obtaining estimation information of the at least one tooth to be estimated.

An aspect of the present application provides a computer-implemented method for measuring a tooth pose difference, including: obtaining first and second three-dimensional digital models, which are two different three-dimensional digital models representing the same dentition; for each pair of corresponding teeth of the first and second three-dimensional digital models, performing coarse registration between them based on their local coordinate systems; performing fine registration for each pair of teeth that have been coarsely registered by using an Iterative Closest Point (ICP) method; selecting a plurality of reference points for each tooth of the first three-dimensional digital model to obtain a first reference point set; based on a result of the fine registration, projecting the reference points of the tooth of the first three-dimensional digital model to a corresponding tooth of the second three-dimensional digital model to obtain a second reference point set, the reference points in the first and second reference point sets forming pairs of reference point pairs; based on the first and second reference point sets, performing overall registration between the first and second three-dimensional digital models; based on the first and second three-dimensional digital models after the overall registration; calculating a position difference of a tooth to be estimated between the first and second three-dimensional digital models; establishing a reference coordinate system for the tooth to be estimated. An extension direction of a z-axis of the reference coordinate system is consistent with a normal direction of an occlusal surface of the dentition, and an extension direction of an x-axis of the reference coordinate system is consistent with an extension direction of a tangent line at a point on a dental arch curve of the dentition corresponding to the tooth to be estimated. The pose difference is decomposed in the reference coordinate system to obtain translation components along the x, y and z axes of the reference coordinate system and rotation components around the x, y and z axes of the reference coordinate system.

In some embodiments, the coarse registration is performed according to an SVD method.

In some embodiments, the coarse registration is performed by: for each pair of teeth, selecting a plurality of pairs of reference points in local coordinate systems of two teeth, each pair of corresponding reference points having same coordinate values, and performing the coarse registration between the pair of teeth based on the plurality of pairs of reference points.

In some embodiments, a weight of a point pair on which the fine registration is based is assigned according to at least one of following: (1) assigning the weight are assigned according to a long axis coordinate of the local coordinate system, a reference point pair closer to an incisal edge or occlusal surface of the tooth having a higher weight, and a reference point pair closer to a gum line having a lower weight; (2) assigning the weight according to reference point pairs sorted by distance; in each iteration, the reference point pairs being sorted from large to small by distance, and a reference point pair with a larger distance having a lower weight; and (3) assigning the weight according to a distance threshold; in each iteration, based on that a distance of a point pair exceeds a preset distance threshold, a weight of the point pair being reduced.

In some embodiments, the computer-implemented method for measuring the tooth pose difference further includes: calculating a confidence level of the fine registration based on a proportion of point pairs that have completed the registration.

In some embodiments, for each pair of teeth, a first point in each point pair on which the fine registration is based is a vertex of a tooth in the first three-dimensional digital model of the pair of teeth, and a second point in the point pair is an intersection point of a ray cast from the first point along a normal direction of the first point with a surface of a tooth in the second three-dimensional digital model of the pair of teeth.

In some embodiments, the overall registration is performed based on the SVD method.

In some embodiments, in the overall registration, a weight of a reference point pair is assigned according to at least one of the following. (1) The weight is assigned according to the designed movement amount of each tooth in a treatment step corresponding to the second three-dimensional digital model, and the smaller the designed movement amount of the tooth corresponding to the reference point, the larger the weight. (2) The weight is assigned according to the confidence level of the fine registration, and the higher the confidence level of the fine registration of the tooth corresponding to the reference point, the larger the weight. (3) The weight is assigned according to a degree of difficulty in tooth movement, and the easier the movement of the tooth corresponding to the reference point is to achieve, the larger the weight. And (4) the weight is assigned according to the position of the reference point on the tooth, and the closer the position to the crown, the larger the weight.

In some embodiments, the second three-dimensional digital model is a three-dimensional digital model representing a current state of the dentition, and the first three-dimensional digital model is one of a series of successive three-dimensional digital models in an orthodontic treatment plan using a shell-style orthodontic appliance, and the series of successive three-dimensional digital models represent target tooth arrangements of a series of successive treatment steps of the orthodontic treatment plan.

In some embodiments, the first and second three-dimensional digital models are obtained by two scans.

In some embodiments, the first three-dimensional digital model is the one of the series of successive three-dimensional digital models that is closest to the second three-dimensional digital model.

Another aspect of the present application provides a computer-implemented method for calculating an achievement rate of orthodontic treatment, including: using the computer-implemented method for measuring a tooth pose difference to measure and obtain a pose difference of the tooth to be estimated between a three-dimensional digital model representing an initial tooth arrangement of a dentition and a three-dimensional digital model representing a current state of the dentition, recorded as a first pose difference, and decomposing the first pose difference in a reference coordinate system; measuring and obtaining a pose difference of the tooth to be estimated between the three-dimensional digital model representing the initial tooth arrangement of the dentition and a three-dimensional digital model representing the target tooth arrangement of the dentition, recorded as a second pose difference, and decomposing the second pose difference in the reference coordinate system; and dividing a component of the first pose difference by a corresponding component of the second pose difference to obtain an achievement rate of the tooth to be estimated in a corresponding movement direction.

In some embodiments, the target tooth arrangement is a target tooth arrangement for a current treatment step in the orthodontic treatment plan using the shell-style orthodontic appliance.

Another aspect of the present application provides a computer system for measuring a tooth pose difference, which includes a storage device and a processor. The storage device stores a computer program for measuring the tooth pose difference, and when the computer program is run by the processor, the computer-implemented method for measuring the tooth pose difference described above is executed.

An aspect of the present application provides a computer-implemented method for measuring a jaw position difference, including: obtaining first and second three-dimensional digital models, the first and second three-dimensional digital models being two different three-dimensional digital models representing a dento-jaw of the same patient, the first and the second three-dimensional digital models each including a maxillary dentition and a mandibular dentition, and the maxillary dentitions and the mandibular dentitions of the first and the second three-dimensional digital models being in the same predetermined state; performing coarse registration between each pair of teeth in the maxillary portions of the first and the second three-dimensional digital models based on local coordinate systems of the pair of teeth; using an ICP method to perform fine registration between the pair of teeth that has been coarsely registered ; selecting a plurality of reference points for each tooth in the maxillary portions of the first three-dimensional digital models to obtain a first reference point set; based on a result of the fine registration, projecting the plurality of reference points of the tooth in the maxillary portion of the first three-dimensional digital model to a corresponding tooth of the second three-dimensional digital model to obtain a second reference point set, and the plurality of reference points in the first and the second reference point sets form pairs of reference point pair; based on the first and the second reference point sets, performing an overall registration on the maxillary portions of the first and the second three-dimensional digital models; and based on the first and the second three-dimensional digital models of the maxillary portion that have been overall registered, calculating a pose difference between mandibular portions of the first and the second three-dimensional digital models.

In some embodiments, the predetermined state is an occluded state.

In some embodiments, the computer-implemented method for measuring the jaw position difference further includes: calculating the pose difference between the mandibular portions of the first and second three-dimensional digital models based on a relative position relationship between the maxillary portion and the mandibular portion of the first three-dimensional digital model, a relative position relationship between the maxillary portion and the mandibular portion of the second three-dimensional digital model, and the result of the overall registration; determining an occlusal surface and midline based on the first three-dimensional digital model, determining a z-axis of the reference coordinate system based on a normal direction of the occlusal surface, determining a y-axis of the reference coordinate system based on the midline, and an x-axis of the reference coordinate system being perpendicular to the z-axis and y-axis; and decomposing the pose difference of the mandibular portions in the reference coordinate system.

In some embodiments, the computer-implemented method for measuring the jaw position difference further includes: based on the first and second three-dimensional digital models with the maxillary portions that have been overall registered, calculating a position difference between selected key points of the mandibular portions of the first and second three-dimensional digital models; and calculating the pose difference between the mandibular portions based on the position difference of the selected key points.

In some embodiments, the coarse registration is performed based on an SVD method.

In some embodiments, the coarse registration is performed by: for each pair of teeth, selecting a plurality of pairs of reference points in the local coordinate systems of the two teeth, each pair of reference points has same coordinate values, and the coarse registration of the pair of teeth is performed based on the pair of reference points.

In some embodiments, a weight of a reference point pair on which the fine registration is based are assigned according to at least one of the following. (1) The weight is assigned according to a long axis coordinate of the local coordinate system, a reference point pair closer to an incisal edge or occlusal surface of the tooth has a higher weight, and a reference point pair closer to a gum line has a lower weight. (2) The weight is assigned according to reference point pairs sorted by distance: and in each iteration, the reference point pairs are sorted from large to small by distance, and a reference point pair with a larger distance has a lower weight. And (3) The weight is assigned according to a distance threshold, and in each iteration, based on that a distance of a reference point pair exceeds a preset distance threshold, a weight of the reference point pair is reduced.

In some embodiments, the computer-implemented method for measuring the jaw difference further includes: calculating a confidence level of the fine registration based on a proportion of point pairs that have completed the registration.

In some embodiments, for each pair of teeth, a first point in each point pair on which the fine registration is based is a vertex of a first tooth in the pair of teeth, and a second point in the point pair is an intersection point of a ray cast from the first point along a normal direction of the first point with a surface of the second tooth in the pair of teeth.

In some embodiments, the overall registration is based on an SVD method.

In some embodiments, in the overall registration, a weight of the reference point pair is assigned according to at least one of the following. (1) The weight is assigned according to a designed movement amount of each tooth in the maxillary portion of the second three-dimensional digital model, and the smaller the designed movement amount of the tooth corresponding to the reference point, the larger the weight. (2) The weight is assigned according to a confidence level of the fine registration, and the higher the confidence level of the fine registration of the tooth corresponding to the reference point, the larger the weight. (3) The weight is assigned according to the degree of difficulty in tooth movement, and the easier the movement of the tooth corresponding to the reference point is to achieve, the larger the weight. and (4) The weight is assigned according to the position of the reference point on the tooth, and the closer the reference point to the crown, the larger the weight.

In some embodiments, the second three-dimensional digital model is a three-dimensional digital model representing the patient's current dento-jaw, and the first three-dimensional digital model is one of a series of successive three-dimensional digital models in an orthodontic treatment plan using a shell-style orthodontic appliance, and the series of successive three-dimensional digital models represent target tooth arrangements of a series of successive treatment steps of the orthodontic treatment plan.

In some embodiments, the first three-dimensional digital model is the one of the series of successive three-dimensional digital models that is closest to the second three-dimensional digital model.

Another aspect of the present application provides a computer system for measuring a jaw difference, which includes a storage device and a processor. The storage device stores a computer program for measuring the jaw difference. When the computer program is run by the processor, the processor will execute the computer-implemented method for measuring the jaw difference.

**Brief Description of Figures**

FIG. 1 is a schematic flow chart of a tooth deviation estimation method executed by a computer in an embodiment of the present application.
FIG. 2A-FIG. 2B are schematic diagrams of tooth registration results in an embodiment of the present application.
FIG. 3A-FIG. 3B are schematic diagrams of tooth registration results in an embodiment of the present application.
FIG. 4A-FIG. 4C are schematic diagrams of tooth deviation estimation in an embodiment of the present application.
FIG. 5A-FIG. 5D are interactive schematic diagrams of a method for estimating a tooth deviation in an embodiment of the present application.
FIG. 6A-FIG. 6C are interactive schematic diagrams of a method for estimating a tooth deviation in an embodiment of the present application.
FIG. 7A-FIG. 7C are schematic diagrams of a method for estimating a tooth deviation in an embodiment of the present application.
FIG. 8 is a schematic structural diagram of a system for estimating a tooth deviation in an embodiment of the present application.

**Detailed Description**

During orthodontic treatment, it is often necessary to evaluate the progress of the treatment plan on a digital model.

A possible implementation method is to compare multiple digital models that were obtained at different times and need to be converted to a unified coordinate system through registration in order to be effectively evaluated. A common method usually relies on manual adjustment to complete the registration, which has the disadvantages of low efficiency, susceptibility to subjective factors, and difficult to control errors.

The present application provides a single-tooth registration and multi-tooth registration solution. Based on a tooth registration algorithm and combined with the actual situation of tooth registration, it delivers results that are efficient, stable, with built-in confidence level, and consistent with clinical cognition.

An application scenario of the method of the present application is that during orthodontic treatment using a shell-style orthodontic appliance, a three-dimensional digital model (i.e., an analysis model) representing the patient's dentition (maxillary dentition or mandibular dentition) in its current state is compared with one of three-dimensional digital models representing multiple successive tooth arrangements of the dentition in the orthodontic treatment plan (i.e., a reference model), and the pose differences of each tooth between the analysis model and the reference model are measured to evaluate the treatment effect based on the measurement results.

Another application scenario of the method of the present application is that after the orthodontic treatment is completed, the three-dimensional digital model (i.e., the analysis model) representing the patient's dentition (maxillary dentition or mandibular dentition) in the current state is compared with a three-dimensional digital model (i.e., the reference model) representing a target tooth arrangement (i.e., the tooth arrangement desired to be achieved by orthodontic treatment) of the dentition in the orthodontic treatment plan, and the pose differences of each tooth between the analysis model and the reference model are measured to determine whether the patient's tooth arrangement after the treatment deviates from the target tooth arrangement based on the measurement results.

It can be understood that the method of the present application is not limited to the above two application scenarios. It is applicable to any application scenario of measuring any tooth pose difference between two different three-dimensional digital models representing the same dentition.

The following takes the first application scenario as an example.

As is known to those skilled in the art, orthodontic treatment of a dentition (maxillary or mandibular dentition) using a shell-style orthodontic appliance usually requires dozens of successive shell-style orthodontic appliances, each shell-style orthodontic appliance corresponding to a treatment step, which is used to reposition the dentition from the tooth arrangement achieved in the previous treatment step to the target tooth arrangement of the current treatment step.

Such an orthodontic treatment plan includes a series of successive three-dimensional digital models, each representing the dentition under the target tooth arrangement for a respective step in the series of successive treatment steps. In an embodiment, the series of successive three-dimensional digital models may be obtained as follows. First, before orthodontic treatment, a three-dimensional digital model representing an original tooth arrangement of the patient is obtained by intraoral scanning or scanning a physical model (such as a plaster model) or an impression of the patient's dentition. Next, the three-dimensional digital model representing the original tooth arrangement is operated to obtain a three-dimensional digital model representing a target tooth arrangement of orthodontic treatment. Then, based on the three-dimensional digital model representing the original tooth arrangement and the three-dimensional digital model representing the target tooth arrangement of orthodontic treatment, three-dimensional digital models representing a series of successive tooth arrangements between them are generated. Since the three-dimensional digital models representing target tooth arrangements of the series of successive treatment steps are generated based on the three-dimensional digital model representing the original tooth arrangement, these three-dimensional digital models may be in the same world coordinate system.

In an embodiment, a three-dimensional digital model representing the patient's dentition in the current state may be obtained by intraoral scanning or scanning a physical model (such as a plaster model) or an impression of the patient's dentition. Since the first and second three-dimensional digital models are obtained by two scans, the two may be in different world coordinate systems. In addition, the morphology of the patient's teeth may change during orthodontic treatment (for example, due to factors such as wear or growth). Therefore, the two need to be registered before comparing them.

As known to those skilled in the art, in some cases, after wearing the first N successive shell-style orthodontic appliances in sequence, the patient may not be able to achieve the target tooth arrangement of the $N^{th}$ treatment step. If the discrepancy between the tooth arrangement and the target tooth arrangement is too large, it can be considered that the actual treatment has deviated from the original treatment plan, and it can be considered to restart the treatment, that is, redesign the treatment plan based on a current tooth arrangement of the patient. N is a positive integer greater than 1.

In an embodiment, the first three-dimensional digital model is a reference model, which serves as a reference for comparison, and the second three-dimensional digital model is an analysis model, which serves as a comparison object. The method of the present application may be used to measure the pose difference of each tooth between the analysis model and the reference model.

The first and second three-dimensional digital models are two different three-dimensional digital models representing the same dentition, and the tooth arrangements and/or tooth morphologies represented by the two models may be different.

Due to the need for subsequent processing, the first and second three-dimensional digital models are three-dimensional digital models that have been segmented, that is, the teeth thereof are independent of each other.

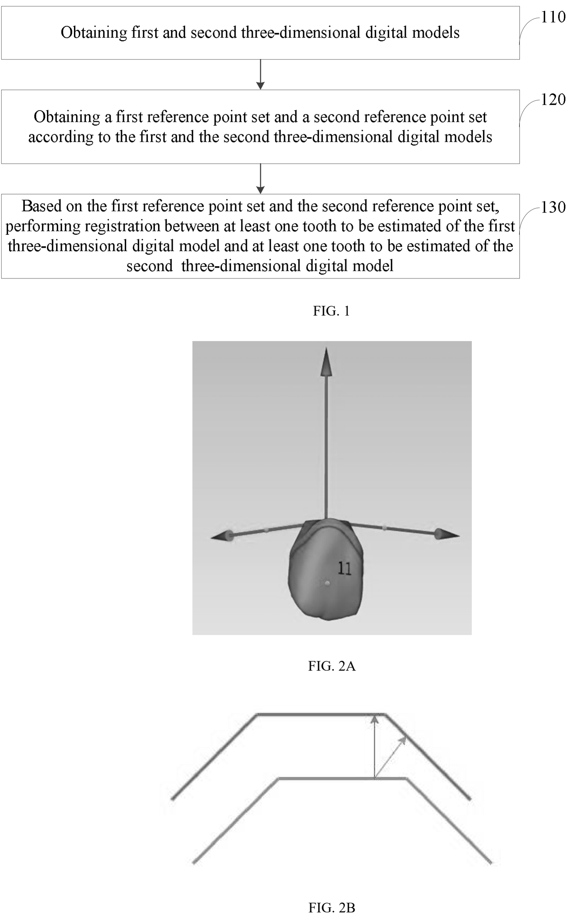

As shown in FIG. 1, the present application provides a tooth deviation estimation method, including following steps.

S110: obtaining first and second three-dimensional digital models.

In S110, the first and second three-dimensional digital models represent different three-dimensional digital models of the same patient; the second three-dimensional digital model is a three-dimensional digital model obtained by collecting teeth of the patient, and the first three-dimensional digital model is one of a series of successive three-dimensional digital models in a tooth orthodontic treatment plan using a shell-style orthodontic appliance.

S120: obtaining a first reference point set and a second reference point set according to the first and the second three-dimensional digital models.

In S120, the first reference point set includes a reference point of at least one tooth to be estimated in the first three-dimensional digital model; and the second reference point set includes a reference point of at least one tooth in the second three-dimensional digital model corresponding to the reference point of the first reference point set.

S130: based on the first reference point set and the second reference point set, performing registration between the at least one tooth to be estimated of the first three-dimensional digital model and the at least one tooth to be estimated of the second three-dimensional digital model.

In S130, a registration result is used to estimate a deviation of the at least one tooth to be estimated.

Optionally, based on the registration result of the at least one tooth to be estimated in the first and second three-dimensional digital models, estimation information of the at least one tooth to be estimated can be obtained.

The digital dental models of the same patient obtained two or more times at different times are registered to provide a reference for subsequent diagnosis, estimation, and treatment plan modification. The registration process is automated and does not require human intervention. In the registration of multiple teeth, other factors of the teeth can also be considered comprehensively, such as the degree of difficulty in tooth movement, the amount of tooth movement, the wearing of orthodontic appliances, and other clinical factors, so that the registration results and estimation information obtained are in line with reality and the clinician's cognition.

In some embodiments, the present application provides a registration method for a single tooth.

The input data may include: an original tooth model of a single tooth, which may be a first three-dimensional digital model (model A); a new tooth model of the single tooth, which may be a second three-dimensional digital model (model B).

The data format may be grid or point cloud data, which is not limited here.

In some embodiments, tooth correspondence information is obtained.

Optionally, a result of the previous processing may be used to ensure that the model A and the model B correspond to the same tooth of the same patient in reality, for example, determined by the FDI number of the tooth.

In some embodiments, tooth pose information is obtained.

Optionally, the local coordinate systems of the model A and the model B may be obtained through the result of the previous processing. For example, an origin of the coordinate system is a center of the tooth, and the three coordinate axes of the coordinate system are a long axis of the tooth, a mesiodistal direction of the tooth, and a labiolingual direction of the tooth.

There are many ways to register a single tooth. The registration may be a single registration or multiple iterative registrations. The registration method may be from model B to model A, or model A to model B, which is not limited here. For the sake of simplicity in description, the following takes the registration of model B to model A as an example. Model B is registered to model A after a rigid transformation (translation and rotation in three-dimensional space).

There are many ways to register, and the following uses point-to-point registration as an example for illustration. The reference point may also be selected in many ways. The following uses methods 1 to 4 as examples for illustration.

Method 1: the registration may be performed based on coordinate point pairs of the local coordinate system.

For example, point pairs used as the registration input are at least three points of model A and model B that have the same coordinate values in their respective local coordinate systems; for example, $(0,0,0)$, $(\pm 1,0,0)$, $(0,\pm 1,0)$, $(0,0,\pm 1)$.

Method 2: the registration may also be performed by using characteristic point pairs of the tooth. For example, the characteristic points on the crown can be identified, and the same characteristic points (at least 3 pairs of characteristic points) on model A and model B can be registered.

Method 3: sample some or all points in one model (model A or model B). For each selected point, a ray is cast along its normal direction to find an intersection point with the other model. A starting point of the ray and the intersection point (if any) are the corresponding point pairs.

Method 4: sample some or all points in one model (model A or model B). For each selected point, find the nearest point in the other model to it, and these two points are the corresponding point pair.

As shown in FIG. 2A, the number 11 on the tooth model represents the FDI number of the tooth, which is used to find a corresponding tooth; the three arrows in red, green, and blue represent the three axes of the tooth coordinate system; the three orange points represent the three points used for coarse alignment, and their coordinates are $(5,0,0)$, $(0,5,0)$, and $(0,0,-5)$, respectively.

As shown in FIG. 2B, the orange and green line segments represent the surfaces of the two models that need to be registered; for a point on the green model, a corresponding point on the orange model can be found according to the normal direction (red arrow) perpendicular to the model or the closest distance (blue arrow) to the orange model.

In another possible implementation, multiple registrations can be performed based on a registration iteration method.

Optionally, the selection of a reference point pair may include multiple methods, which may refer to the above-mentioned methods 1 to 4, or other methods, which are not limited here.

Optionally, when a calculated distance of a point pair satisfies a distance threshold condition, the point pair is considered to be fully registered. The threshold condition may be less than a preset threshold, for example, the threshold may be determined by referring to the accuracy of the instrument for obtaining the digital dental model, such as 0.1 or 0.2 mm. The threshold condition may also be set in other ways, which are not limited here.

Optionally, the iteration condition may be determined to be satisfied based on the proportion of fully registered point pairs. The iteration is stopped based on the proportion of fully registered point pairs satisfying a proportion threshold condition, for example, when the proportion of fully registered point pairs in all point pairs is greater than this value.

Optionally, the iteration condition may be determined to be satisfied based on the maximum number of iteration steps. The number of iteration steps may satisfy the maximum number of iteration steps. For example, when the number of iteration steps reaches the maximum number of iteration steps, the iteration is stopped.

Optionally, the iteration condition may be determined to be satisfied based on the minimum update difference. When the minimum update difference satisfies an update threshold condition, the iteration is stopped. For example, when a difference between a pose after the current iteration and a pose after the previous iteration is less than a given threshold (comprehensively evaluated by the translation amount and the rotation amount), the iteration is stopped.

Optionally, when the iteration of registration stops, the registration result of model A and model B and a corresponding rigid transformation (translation and rotation in three-dimensional space) are obtained.

Optionally, the registration result may be determined based on a confidence level. When a registration confidence level meets a confidence level threshold condition, the iteration is stopped. There are many ways to determine the confidence level. For example, the proportion of the registration point pairs may be calculated under a final registration result; for example, the above-mentioned "proportion of completely registered point pairs" is used as the basis for the confidence level. The higher the confidence level, the more reliable the registration result. When the confidence level is greater than a threshold, the registration result is used as the final registration result and is output as a reference for subsequent processing.

Considering that although the model A and the model B correspond to the same tooth, their digital models often cannot completely overlap due to tooth wear, attachments during treatment, changes in the gum line, etc., the registration method needs to remove the influence of these parts that should not overlap as much as possible. Optionally, at least one of following methods can be used.

A weight is assigned according to a long axis coordinate. The weight close to an incisal edge and occlusal surface of the tooth are higher, and the weight close to the gum is lower, so as to reduce the interference of changes in the gum line.

A weight is assigned according to distance sorting. In each iteration, point pairs are sorted from large to small by distance, and weights of some point pairs with larger distances are reduced.

A weight is assigned according to a distance threshold. In each iteration, based on that the distance of a point pair exceeds a certain threshold, a weight of the point pair is reduced. The weight of the point pair may even be reduced to zero, i.e., the point pair does not participate in the registration of the current iteration.

Based on the above method, outlier information may be output. For example, in the final registration result, a point pair without a corresponding relationship is calculated. For example, compared with the above "distance threshold"; the point pair without the corresponding relationship is output as a morphological difference between model A and model B.

It should be noted that after a reference point pair is selected, there can be multiple registration algorithms for the reference point pair, and the registration may be performed by a Singular Value Decomposition (SVD) method as exemplified above, or by other registration methods, which are not limited here. For example, an Iterative Closest Point (ICP) method may be used to register model A and model B, and iterate until the stop condition is reached.

It should be noted that there may be various registration algorithms, and the registration may be performed by the ICP method or by other iterative registration methods, which are not limited here.

In another possible implementation, model A and model B may be registered by multiple registrations based on a single registration result, and iterated until a stop condition is reached. The specific iteration method may refer to the above implementation, which will not be described in detail here.

Of course, model A and model B may also be registered by combining single registration and iterative registration, or by combining a selection method of a reference point. The specific combination method may be determined with reference to the actual application scenario and is not limited here.

For example, coarse registration and fine registration may be included.

For each pair of corresponding teeth in the first and second three-dimensional digital models, they are coarsely registered based on their local coordinate systems; the coarse registration includes: for each pair of teeth, a plurality of reference points that are in a one-to-one correspondence are selected in the local coordinate systems of the two teeth, each pair of corresponding reference points has the same coordinate value, and the coarse registration of the pair of teeth is based on these reference points.

Performing fine registration between each pair of teeth that have been coarsely registered; based on a result of the fine registration, projecting a reference point of each tooth of the first three-dimensional digital model to a corresponding tooth of the second three-dimensional digital model to obtain a second reference point set. For example, for each pair of teeth, a first point in each point pair on which the fine registration is based is a vertex of the tooth of the first three-dimensional digital model in the pair of teeth, and a second point in the point pair is an intersection point of a ray casted from the first point along its normal direction with a facet of the tooth of the second three-dimensional digital model in the pair of teeth.

The present application also provides a registration solution for multiple teeth.

Optionally, the input data may include: tooth model data of at least one tooth.

For example, an original three-dimensional digital model A of multiple teeth (may include: model $A^1$, ..., $A^n$) may be included, and a new three-dimensional digital model B of multiple teeth (may include: model $B^1$, ..., $B^n$) may also be included, and the superscript represents a tooth number.

Optionally, the input data may include: step information of the tooth model. For example, the step information may include: the original three-dimensional digital model A in each step corresponding to the original three-dimensional digital model (model $A^1_1$, ..., $A^1_n$, ..., $A^m_1$, ..., $A^m_n$), where the subscript represents the step information.

Optionally, the input data may include: a correspondence of at least one tooth to be registered, to ensure a correct correspondence. For example, the correspondence is determined by the FDI number, and may also be determined by other methods, which are not limited here.

Optionally, the input data may include: multiple teeth to be registered. For example, the multiple teeth are usually selected as all teeth of a single jaw, or multiple teeth of both jaws, which is not limited here.

The method may also include: calculating registration of a single tooth, and obtaining a registration result of the single tooth, including at least one of the following: each pair of tooth models are located in the same coordinate system after registration; a confidence level of a final registration result of each pair of teeth; and abnormal points under the final registration result of each pair of teeth.

Optionally, the input data may include: the step number k corresponding to the last pair of orthodontic appliances worn by the patient when the new three-dimensional digital model is established.

Optionally, the input data may include: the degree of difficulty in tooth movement in orthodontic design.

In a possible implementation, the registration result with at least one tooth to be estimated may be determined based on the tooth reference point information. Thereby, based on the registration result, it is determined to perform deviation analysis on the step scheme.

Optionally, the tooth reference point information may be determined in various ways.

For example, one or more reference points may be selected on model A based on each tooth involved in the registration; for example, the reference point may be a tooth morphological feature point, a buccal cusp point, a Functional Axis (FA) point, an adjacent point, etc.; for another example, the reference point may be a fixed point $(\pm3,0,0)$, $(0,\pm3,0)$, $(0,0,\pm3)$ in the coordinate system. Based on the reference point that is selected, the tooth reference point information corresponding to the reference point may be determined. The tooth reference point information can include tooth feature point information, tooth coordinate information, and other information, which can be determined based on the specific implementation process and is not limited here.

Optionally, tooth reference point projection information may include various forms. The projection information may be coordinate information, and may also include other data, which may be determined based on a specific implementation process and is not limited here.

For example, for each tooth, the registration result of a single tooth may be used to project a reference point on model A to model B. Of course, a reference point on model B may also be projected to model A. Other projection methods may also be used to determine, which are not limited here.

Optionally, the tooth reference point information may also include weight information. There are many ways to determine the weight information. The following examples are provided.

For example, the weight information may be determined based on at least one of the following:
it may be assigned according to a preset weight; the setting method is not limited.

Optionally, it is determined according to a registration result of a single tooth. For example, it is determined according to the confidence level of the registration result, for example, a higher confidence level corresponds to a larger weight.

Optionally, it may also be determined according to the degree of difficulty in tooth movement. For example, a movement that is easier to achieve corresponds to a larger weight.

Optionally, it may also be determined according to the designed amount of step movement of the tooth. For example, a smaller designed movement amount has a larger weight.

Optionally, it may also determine according to a relative position of a reference points on the tooth, for example, a point closer to the crown and farther from the gum may have a greater weight.

Optionally, a plurality of registration methods may be used to determine a registration tooth and a registration result for at least one tooth to be estimated.

The tooth model to be registered may include at least one tooth model, and the registration result is determined based on the at least one tooth model.

Optionally, the tooth model to be registered may be determined based on the obtained step information.

In a possible implementation, the step information includes at least one step number.

The step information may be step by step information specified by a user or may be estimated step information obtained based on the registration.

In another possible implementation, a differences between models $B_1$, ..., $B_n$ and model A can be calculated, and a step corresponding to model B having the smallest difference is selected as the step information of the second three-dimensional digital model to be estimated.

For example, the step information may be the step number k corresponding to the last pair of orthodontic appliances worn by a given patient. Based on the step number k, the tooth model $A_k$ corresponding to the step number is determined.

After the patient wears the first N successive shell-style orthodontic appliances, a target tooth arrangement of the $N^{th}$ treatment step may not be achieved, but may be closer to a target tooth arrangement of a treatment step before the $N^{th}$ treatment step. Therefore, in an embodiment, the second three-dimensional digital model may be registered and compared with the three-dimensional digital models representing the target tooth arrangements of the multiple successive treatment steps one by one to find the closest one.

Optionally, within a certain range (e.g., 5 steps or 10 steps) before the step number k, based on the step information, determine the tooth model A corresponding to the step information. For example, the tooth model A may include tooth models $A_{k-5}$ to $A_k$. The tooth model A may include tooth models $A_{k-6}$ to $A_{k-1}$. Other methods are also possible and are not limited here.

For example, the step number in the step information may also be a step interval. For example, based on the step information corresponding to the last pair of orthodontic appliances worn by a given patient, all step positions within a certain range (for example, 5 steps or 10 steps) before this step information may be selected as the step interval.

The tooth model to be registered hereinafter may include at least one tooth model corresponding to each step. For the convenience of description, the following takes a tooth model corresponding to a step as an example. For example, tooth model A includes tooth models $A^1$, ..., $A^n$ of n teeth. Tooth model B includes tooth models $B^1$, ..., $B^n$ of n teeth.

There are many registration methods, and the following uses methods A to C as examples to illustrate.

Method A, the registration result is determined based on the reference point pairs of the tooth models corresponding to the multiple teeth.

In a possible implementation, the reference points in the tooth model A are combined as a first group of reference points. The coordinate system of the first group of reference points can use the coordinate system of the combined models $A_1$ to $A_n$; the reference points in the tooth model B are combined to form a second group of reference points.

Based on the determined tooth model, the registration result corresponding to each tooth model may be calculated. Optionally, the registration result may be obtained by solving the optimal solution based on an algorithm. The registration result may also include residual information. Optionally, the solution with the smallest residual is the final registration result.

Optionally, the reference point pairs may be registered using the coordinate system of the combined models $B^1$ to $B^n$, or using other coordinate systems, which are not limited here.

In a possible implementation, based on the above-mentioned paired reference point pairs and the set weight, the optimal registration result between the combined models $A^1$ to $A^n$ and the combined models $B^1$ to $B^n$ is solved and a corresponding rigid transformation (translation and rotation in three-dimensional space) and a distance residual of the registered point pairs (weighted sum of squared distances) are obtained.

Method B, based on a probability distribution model, obtaining registration results of multiple teeth.

Optionally, a normal distribution model is established based on position information of reference points of a first model.

According to the normal distribution model data of the first model, distance information between the reference points of the second model and the first model is determined. According to the distance information of each reference point, the distance information of the model is determined. According to the distance information of the model, a registration result is determined.

For example, the first model is model A and the second model is model B.

Optionally, taking the step number k corresponding to the step information as an example, a normal distribution model is established based on a position of the three-dimensional space of the model $A_k$ to obtain point mean and covariance matrix of each reference point in the model $A_k$. The specific process may include each reference point in different tooth models $A^1$, ..., $A^n$.

Optionally, for each reference point in models $B^1$, ..., $B^n$, the distance information of each reference point may be a distance (Mahalanobis distance) between the reference point of model B and the normal distribution model of the above-mentioned model A obtained by calculation.

There are many ways to determine the distance information of the model based on the distance information of each reference point. For example, the distance information of the model can be obtained by calculating the sum of squares of the distances of each reference point. The distance information of the model can be minimized to obtain the final rigid transformation (translation and rotation in three-dimensional space). Optionally, all reference points in the model $B^1$, ..., $B^n$ maintain synchronous rigid transformation.

Method C, obtaining the registration results of multiple teeth based on the average transformation amount.

Weighted processing is performed on the registration result of each tooth to obtain registration reference information of the tooth to be registered.

Optionally, the registration result of each tooth may be obtained by referring to the method in the above embodiments, and the registration result may include a representation of the rigid transformation (translation and rotation in three-dimensional space), and optionally, the registration result may also include a representation of the confidence level.

Optionally, at least one of the translation amounts (three-dimensional vectors) and rotation amounts (quaternions or rotation matrices) of all teeth is weighted and summed according to tooth weight information to obtain weighted information of at least one tooth, which serves as the registration reference information of the tooth to be registered.

The registration result is determined by comparing the registration reference information of the tooth to be registered with the registration information of the single tooth.

Optionally, a difference between at least one of the translation and rotation amounts of each tooth and the weighted summation result is calculated respectively, and the differences are summed (taking the absolute value to keep the value non-negative) to obtain the residual information of the registration result.

In a possible implementation, the registration result is determined according to the residual information of the registration result. Optionally, a registration result with the smallest residual may be used as the registration result of the multiple teeth.

In a possible implementation, the registration result is determined according to a confidence level result.

The confidence level information may be determined in various ways, for example, it may include at least one of the following.

A corresponding confidence level may be determined according to the residual of the registration result. For example, the larger the residual, the lower the confidence level.

A corresponding confidence level may be determined according to a difference between the final step number and the initial step number. In a possible implementation, the confidence level may be determine based on a difference between the estimated step information (or the specified step number) and the corresponding step number when the first three-dimensional digital model is collected. For example, the greater the difference between the final step number and the initial step number (indicating a greater time span between two models), the lower the confidence level.

A corresponding confidence level may be determined according to the sum of squares of the Mahalanobis distances of the registration results. For example, the larger the value of the sum of the squares, the lower the confidence level.

Optionally, the registration result may include: corresponding rigid transformation (translation and rotation in three-dimensional space) information between the models.

Optionally, the registration result may include: confidence level information.

Optionally, the registration result may include: estimated step information; and estimated step numbers is used to represent a step position that is closest to the actual state of the patient's teeth.

In a possible implementation, at least one of the translation and rotation amounts corresponding to each step number is calculated according to the step number included in the step information, and the corresponding residual may also be calculated. Optionally, the step number corresponding to the smallest residual is used as the estimated step information.

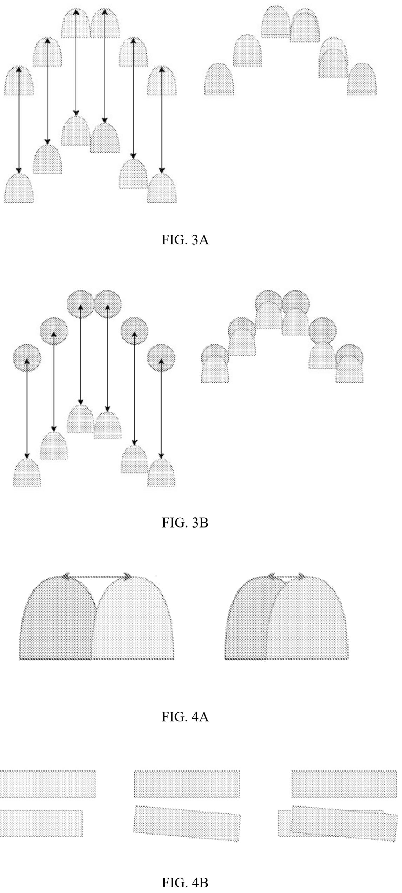

As shown in FIG. 3A, orange and green represent two multi-tooth models that need to be registered; the black arrow on the left represents a corresponding relationship between the reference points of each tooth, which comes from the result of single tooth registration; an overlapping image of the two models is shown on the right, which indicates the registration result obtained after using the registration method of at least one tooth to the above-mentioned corresponding points.

As shown in FIG. 3B, the green model on the left represents a model that needs to be registered, in which a reference point is a position pointed by an arrow; in another model, a normal distribution is established for each reference point, represented by a red circle, and a center of the circle represents a position with the highest probability; the registration result is shown on the right side, and the reference point (highest point) of the green model roughly coincides with the center of the red circle.

In the embodiments of the present application, the tooth number and the local coordinate system may be used to coarsely register a single tooth. The ICP method may also be used in combination with characteristics of tooth morphology to finely register a single tooth. Optionally, characteristics of tooth movement may be combined with step information in the treatment plan to further calculate a registration result of multiple teeth (such as single-jaw teeth) from a registration result of the single tooth. The specific method is not limited. Optionally, in each step of the registration, a confidence level is introduced to identify the accuracy and credibility of the results.

The digital dental models of the same patient obtained two or more times at different times are registered to provide a reference for subsequent diagnosis, estimation, and treatment plan modification. The registration process is automated and does not require human intervention. The registration results are automatically estimated through confidence levels. A few cases with low confidence levels are automatically discovered to facilitate separate processing. In the registration of multiple teeth, clinical factors such as the degree of difficulty in tooth movement, the amount of tooth movement, and the wearing of orthodontic appliances are comprehensively considered, and the results obtained are consistent with reality and the cognition of clinicians.

On the one hand, the present application provides a computer-implemented method for measuring a tooth pose difference, which is used to measure pose differences of teeth to be estimated between two three-dimensional digital models representing the same dentition. The tooth pose includes the position and posture (i.e., angle) of the tooth.

On the other hand, the present application provides a computer system for measuring tooth pose difference, which includes a storage device and a processor. The storage device stores a computer program for a measuring tooth pose difference. When the computer program is run by the processor, the method for measuring the tooth pose difference is executed.

The computer-implemented method for measuring a tooth pose difference in an embodiment of the present application includes the following steps.

In step 101, obtaining first and second three-dimensional digital models.

The first and second three-dimensional digital models are two different three-dimensional digital models representing the same dentition, and tooth arrangements and/or tooth morphologies represented by the two models may be different.

In an embodiment, the first three-dimensional digital model is a reference model, which serves as a reference for comparison, and the second three-dimensional digital model is an analysis model, which serves as a comparison object. The method of the present application may be used to measure the pose difference of each tooth between the analysis model and the reference model.

An application scenario of the method for measuring the tooth pose difference of the present application is that during orthodontic treatment using a shell-style orthodontic appliance, a three-dimensional digital model (i.e., an analysis model) representing the patient's dentition (maxillary dentition or mandibular dentition) in the current state is compared with a selected one of multiple three-dimensional digital models representing multiple successive tooth arrangements of the dentition in the orthodontic treatment plan (i.e., a reference model), and the pose differences of each tooth between the analysis model and the reference model are measured to evaluate the treatment effect based on the measurement results.

Another application scenario of the method for measuring the tooth pose difference of the present application is that after the orthodontic treatment is completed, the three-dimensional digital model (i.e., the analysis model) representing the patient's dentition (maxillary dentition or mandibular dentition) in the current state is compared with a three-dimensional digital model (i.e., the reference model) representing the target tooth arrangement(i.e., the tooth arrangement hoped to be achieved by orthodontic treatment) of the dentition in the orthodontic treatment plan, and the pose differences of each tooth between the analysis model and the reference model is measured to determine whether the patient's tooth arrangement after the treatment deviates from the target tooth arrangement based on the measurement results.

It can be understood that the method for measuring the tooth pose difference of the present application is not limited to the above two application scenarios. It is applicable to any application scenario of measuring any tooth pose difference between two different three-dimensional digital models representing the same dentition.

Taking the first application scenario mentioned above as an example, the method for measuring the tooth pose difference of the present application is described in detail below.

As is known to those skilled in the art, orthodontic treatment of a dentition (maxillary or mandibular dentition) using a shell-style orthodontic appliance usually requires dozens of successive shell-style orthodontic appliances, each shell-style orthodontic appliance corresponding to a treatment step, which is used to reposition the dentition from the tooth arrangement achieved in the previous treatment step to the target tooth arrangement of the current treatment step.

Such an orthodontic treatment plan includes a series of successive three-dimensional digital models, each representing the dentition under the target tooth arrangement for a respective step in the series of successive treatment steps. In an embodiment, the series of successive three-dimensional digital models may be obtained as follows. First, before orthodontic treatment, a three-dimensional digital model representing an original tooth arrangement of the patient is obtained by intraoral scanning or scanning a physical model (such as a plaster model) or an impression of the patient's dentition. Next, the three-dimensional digital model representing the original tooth arrangement is operated to obtain a three-dimensional digital model representing a target tooth arrangement of orthodontic treatment. Then, based on the three-dimensional digital model representing the original tooth arrangement and the three-dimensional digital model representing the target tooth arrangement of orthodontic treatment, three-dimensional digital models representing a series of successive tooth arrangements between them are generated. Since the three-dimensional digital models representing target tooth arrangement of the series of successive treatment steps are generated based on the three-dimensional digital model representing the original tooth arrangement, these three-dimensional digital models are in the same world coordinate system.

In an embodiment, a three-dimensional digital model representing the patient's dentition in the current state may be obtained by intraoral scanning or scanning a physical model (such as a plaster model) or an impression of the patient's dentition. Since the first and second three-dimensional digital models are obtained by two scans, they may be in different world coordinate systems. In addition, the morphology of the patient's teeth may change during orthodontic treatment (for example, due to factors such as wear or growth). Therefore, the two need to be registered before comparing them.

As known to those skilled in the art, in some cases, after the patient wears the first N successive shell-style orthodontic appliances in sequence, the patient may not be able to achieve a target tooth arrangement of the $N^{th}$ treatment step, but may be closer to a target tooth arrangement of a treatment step before the $N^{th}$ treatment step. Therefore, in an embodiment, the second three-dimensional digital model may be registered and compared with three-dimensional digital models representing target tooth arrangements of multiple successive treatment steps one by one to find the closest one. If a difference in the number of steps between a treatment step found and a treatment step last completed by the patient is too large (for example, greater than a preset step number difference threshold), it can be considered that the actual treatment has deviated from the original treatment plan, and it can be considered to restart the treatment, that is, redesign a treatment plan based on a current tooth arrangement of the patient.

Due to the need for subsequent processing, the first and second three-dimensional digital models are three-dimensional digital models that have been segmented, that is, the teeth thereof are independent of each other.

In step 103, performing coarse registration between corresponding teeth in the first and second three-dimensional digital models.

In an embodiment, the teeth in the first and second three-dimensional digital models may be numbered in a predetermined manner, and the teeth in the first and second three-dimensional digital models may be paired in pairs according to tooth numbers (i.e., the same tooth may be found in the first and second three-dimensional digital models).

In order to facilitate calculation, in addition to setting a world coordinate system for a three-dimensional digital model of a dento-jaw, a local coordinate system is also set for the three-dimensional digital model of each tooth.

Since setting the local coordinate system using current technology (e.g., a local coordinate system setting method based on deep learning) may achieve extremely high accuracy and consistency, in an embodiment, two three-dimensional digital models of the same tooth may be coarsely registered based on the local coordinate system.

In an embodiment, for the three-dimensional digital model of the same tooth in the first and second three-dimensional digital models, at least three points in its local coordinate system may be selected as reference points, and the two three-dimensional digital models of the tooth may be coarsely registered based on these reference points. For example, the four points (0,0,0), (1,0,0), (0,1,0) and (0,0,1) may be used as the reference points. It is understood that the selection of reference points is not limited to this example, as long as they are not on the same straight line.

In some cases, there may be differences between the three-dimensional digital models of the same tooth in the first and second three-dimensional digital models. For example, tooth wear, addition/removal of attachments, or changes in the gum line (for example, which may be caused by the growth of erupted teeth, vertical movement or tilt of teeth, etc.) may cause a difference between the three-dimensional digital models of the same tooth scanned at different times.

If there are large differences between the two three-dimensional digital models of the same tooth, for example, a morphological difference between the three-dimensional digital models obtained by scanning at different time points during the tooth eruption process may be large, and the two three-dimensional digital models may not be well coarsely registered based on the local coordinate system. In this case, feature points may be used as reference points for registration, such as buccal cusp points, FA points, and adjacent points. At present, there are many methods for identifying feature points on three-dimensional digital models of teeth, such as a feature point recognition method based on deep learning. The recognition of feature points will not be described in detail here.

In an embodiment, two three-dimensional digital models of the same tooth may be measured, for example, the mesiodistal width and crown height of the tooth, and the measured difference and a preset threshold may be compared to determine whether there is a large difference between the two three-dimensional digital models of the same tooth. If there is a large difference, the feature points are used as reference points for coarse registration, otherwise, coarse registration is performed based on the local coordinate system.

In an embodiment, the corresponding teeth in the first and second three-dimensional digital models may be coarsely registered in pairs based on the reference points using a Singular Value Decomposition (SVD) method.

In step 105, based on a coarse registration result, performing fine registration between corresponding teeth of the first and second three-dimensional digital models.

After the coarse registration, each tooth of the first three-dimensional digital model is coarsely registered with a corresponding tooth of the second three-dimensional digital model. On this basis, these teeth can be finely registered in pairs.

In an embodiment, an iterative closest point algorithm (hereinafter referred to as ICP algorithm) may be used to finely register two three-dimensional digital models of the same tooth.

In an embodiment, two three-dimensional digital models of the same tooth may be finely registered based on a vertex-to-surface approach. For ease of description, the two three-dimensional digital models of the same tooth are hereinafter referred to as model A and model B, respectively.

In an embodiment, point pairs for the fine registration may be determined according to the following method. Some vertices sampled or all vertices selected from model A are taken as a first point set for the fine registration. For each point in the first point set, a ray is cast from the point along a normal direction, an intersection point of the ray with model B (i.e., an intersection point of the ray with a surface of model B) is obtained, and a starting point of the ray and the intersection point are taken as a point pair.

Since the relative position relationship between models A and B is unknown, the intersection point of a unidirectional ray with model B is not necessarily a valid intersection point. Therefore, rays may be cast from a vertex on model A along the normal direction in two opposite directions, or a straight line may be drawn passing through the vertex on model A. In this way, two intersection points with model B may be obtained, and the closer intersection point is selected.

In addition, model B may lack a portion which is in model A. Therefore, a threshold may be set. If the distance between a vertex and each corresponding intersection point is greater than the threshold, it is considered that the rays from the vertex along the normal direction have no valid intersection point with model B.

In yet another embodiment, two three-dimensional digital models of the same tooth may be finely registered based on a vertex-to-vertex approach.

In an embodiment, the point pairs for the fine registration may be determined according to the following method: some vertices sampled or all vertices selected from model A are taken as a first point set for registration. For each point in the first point set, a vertex on model B closest to the point is found, and the two vertices are taken as a point pair.

In an embodiment, a distance threshold may be set. During the iteration process, if the distance of a point pair is less than the distance threshold, the point pair is considered to have completed the registration. In an embodiment, the distance threshold may be determined based on the accuracy of a scanning device used to generate the first and/or second three-dimensional digital models. For example, if the accuracy of the scanning device used is 0.1 mm, then the distance threshold may be set to 0.1 mm, or 0.08 mm, or 0.12 mm, etc. It may be changed according to actual conditions. For example, when the device ensures that the modeling accuracy of a single tooth reaches 0.05 mm, the threshold 1 may be set to 0.05 mm.

In an embodiment, a proportion threshold may be set. If the proportion of the point pairs that have completed the registration is greater than the proportion threshold, it is considered that the registration of the models A and B is completed.

In an embodiment, the following conditions can be set. If any one of these conditions is met, the iteration is stopped: (1) the proportion of point pairs that have completed the registration is greater than the proportion threshold; (2) the number of iterations exceeds a preset iteration number threshold; and (3) the difference between the pose after the current iteration and the pose after the previous iteration is less than a preset pose difference threshold (a comprehensive estimation based on translation and rotation amounts).

Although the models A and B correspond to the same tooth, as mentioned above, due to wear, addition/removal of attachment, and changes in the gum line, the models A and B may not completely overlap. Therefore, it is necessary to minimize the influence of these factors as much as possible during the registration process.

In an embodiment, at least one of the following methods may be used to assign a weight to a point on which the fine registration is based, so as to minimize the influence of the above factors on the fine registration.

(1) A weight is assigned according to a long axis coordinate of the local coordinate system. A point pair closer to the incisal edge or occlusal surface of the tooth has a higher weight, while a point pair close to a gum line has a lower weight, so as to minimize the interference caused by the change of the gum line.

(2) A weight is assigned according to point pairs sorted by distance. In each iteration, point pairs are sorted from large to small by distance, and a point pair with a larger distance has a lower weight, so as to minimize interference caused by morphological differences.

(3) A weight is assigned according to a distance threshold. The distance threshold may be set in advance. For example, the distance threshold may be set based on the scanning accuracy. In each iteration, if the distance of a point pair exceeds the distance threshold, it is considered that the distance of the point pair is caused by morphological differences, and a weight of the point pair is reduced accordingly. The weight of the point pair may even be reduced to zero, i.e., the point pair does not participate in this iteration.

When the iteration of fine registration stops, the following results are output.

(1) A rigid transformation (translation and rotation in three-dimensional space) between the models A and B.

(2) A confidence level: the proportion of point pairs that have completed registration. The higher the proportion, the higher the confidence level, indicating that the registration result is more reliable and can be used as a reference for subsequent processing.

(3) An anomalous point: a point pair(s) that has/have not completed registration when the iteration stops. For example, the distance(s) of the point pair(s) may be compared with the above-mentioned distance threshold, and the point pair(s) with distance(s) greater than the distance threshold is/are regarded as the morphological difference(s) between the models A and B.

In step 107, based on a result of the fine registration, performing overall registration between the first and second three-dimensional digital models.

In an embodiment, a reference point of each tooth in the first three-dimensional digital model may be projected to a corresponding tooth in the second three-dimensional digital model according to the result of the fine registration (the coordinates of the reference point of the second three-dimensional digital model are consistent with the coordinates of the corresponding reference point of the first three-dimensional digital model), and based on the two reference point sets, the first and second three-dimensional digital models are registered as a whole.

In an embodiment, a reference point of each tooth in the first three-dimensional digital model may use a reference point selected by the coarse registration. It is understood that a reference point may also be re-selected for each tooth in the first three-dimensional digital model, and the same method as in the coarse registration may be used to re-select the reference point.

One of the purposes of performing registration between the first and second three-dimensional digital models as a whole is to compare pose differences of the moving teeth between the first and second three-dimensional digital models, to evaluate the actual orthodontic treatment effect based on this. Since the tooth arrangement actually achieved when completing a treatment step is likely to be different from a target tooth arrangement of the treatment step, in this case, the first and second three-dimensional digital models cannot completely overlap as a whole, so it is hoped that the registration will focus on the registration of the stationary teeth to more realistically evaluate the actual orthodontic treatment effect. Therefore, when performing the overall registration, on the one hand, a weight may be assigned according to the designed movement amount of each tooth in the treatment step corresponding to the second three-dimensional digital model, and the smaller the designed movement amount, the greater the weight.

On the other hand, a weight may be assigned according to the confidence level of the registration result of the single tooth, and the higher the confidence level, the greater the weight.

In yet another aspect, a weight may also be assigned according to the degree of difficulty in tooth movement. For movements that are relatively easy to achieve, after completing a treatment step, a pose of the tooth is closer to a target pose of the treatment step, so a larger weight may be assigned to the tooth.

In yet another aspect, a weight may be assigned according to positions of reference points on the teeth, and the closer the reference point is to the crown, the greater the weight.

At least one of the above methods may be used to assign weights to the reference point pairs. After the weights are assigned to the reference points, overall registration may be performed based on these reference points.

In an embodiment, the SVD method may be used to perform overall registration based on the reference points. The obtained results include a rigid transformation and a confidence level.

In an embodiment, the confidence level is determined based on the residual after registration, and the larger the residual, the lower the confidence level.

In yet another embodiment, a normal distribution transformation (NDT for short) method may be used to perform overall registration based on the reference points.

In an embodiment, the sum of the squares of the Mahalanobis distances of the reference points after registration may be used as a confidence level indicator. The larger the value of the sum of the squares, the lower the confidence level.

In yet another embodiment, a weight may be assigned to each tooth, and a weighted average transformation may be calculated and obtained based on the rigid transformation obtained by the fine registration, and the weighted average transformation may be used as the overall registration result of the first and second three-dimensional digital models.

In an embodiment, a weight of each tooth may be set according to at least one of the following. (1) the weight is assigned according a designed movement amount of the tooth (for example, the designed movement amount of a corresponding treatment step, or the total designed movement amount from the original tooth arrangement to a next corresponding treatment step), and the smaller the designed movement amount, the greater the weight; (2) the weight is assigned according to a confidence level of a registration result of a single tooth, and the higher the confidence level, the greater the weight; (3) the weight is assigned according to the degree of difficulty in tooth movement (for example, the movement of a corresponding treatment step, or the movement from the original tooth arrangement to a corresponding treatment step), and the easier the movement is to achieve, the greater the weight.

For each tooth, a difference between the rigid transformation of the fine registration and the weighted average transformation is calculated, and all differences are summed (taking the absolute value to keep the value non-negative) to obtain the residual of the registration. The residual of the registration may be used as an indicator of the confidence level. The higher the residual, the lower the confidence level.

After the first and second three-dimensional digital models are registered, it can be considered that they are in the same world coordinate system. On this basis, the pose difference of each tooth between the first and second three-dimensional digital models may be measured by directly comparing the two.

In many cases, it is not necessary to measure the pose differences of all teeth, but only the pose differences of the teeth to be estimated.

Orthodontic treatment usually only targets teeth with incorrect positions, that is, the teeth that are moved during orthodontic treatment usually only account for a portion of the entire dentition. The estimation of the effectiveness of orthodontic treatment generally only involves the teeth that are moved. In an embodiment, the teeth to be estimated may be teeth that are partially or completely moved. In another embodiment, the teeth to be estimated may be selected by a dental professional, for example, teeth that the dental professional is particularly interested in, or teeth that the dental professional believes may deviate from the target position during the treatment process.

In an embodiment, for each tooth to be estimated, the measured pose difference may include a translation matrix T and a rotation matrix M.

However, the pose difference measured at this time is expressed as the components of each coordinate axis of the world coordinate system, which has little reference value in medicine.

When designing an orthodontic treatment plan, the movement of teeth is usually divided into translation in a mesiodistal direction, translation in a labiolingual direction, translation in a vertical direction, axial tilt, torque, and torsion. In order to make the measured pose difference more medically referential, a reference coordinate system may be established for each tooth to be estimated, and the pose difference of these teeth between the first and second three-dimensional digital models may be decomposed in the corresponding reference coordinate system. The result obtained in this way is more intuitive in medicine, which can make it easier for dental professionals to analyze and evaluate the treatment effect.

In step 109, establishing a reference coordinate system for each tooth to be estimated, and decomposing a pose difference of the tooth to be estimated between the first and second three-dimensional digital models in the reference coordinate system.

In an embodiment, an occlusal surface and dental arch curve may be determined based on the dentition, and the normal direction of the occlusal surface is used as the z-axis of each reference coordinate system, and the tangent of a point on the dental arch curve corresponding to a tooth to be estimated (for example, a point closest to the tooth) is used as the x-axis of the reference coordinate system of the tooth, and the y-axis of each reference coordinate system is perpendicular to the z-axis and the x-axis.

In an embodiment, the occlusal surface may be determined and the dental arch curves may be generated based on the first three-dimensional digital model. In another embodiment, the occlusal surface may be determined and the dental arch curve may be generated based on the second three-dimensional digital model. In another embodiment, the occlusal surface may be determined and the dental arch curve may be generated based on any one of a series of successive three-dimensional digital models in the orthodontic treatment plan. The method for determining the occlusal surface and generating the dental arch curve based on the three-dimensional digital model of the dentition is well known in the industry and will not be repeated here.

In the reference coordinate system of each tooth to be estimated, the x-axis component represents a mesiodistal direction, the y-axis component represents a labiolingual direction, the z-axis component represents a vertical direction, a rotation angle around the x-axis is the torque, a rotation angle around the y-axis is the axial tilt, and a rotation angle around the z-axis is the torsion.

The pose difference of a tooth to be estimated between the first and second three-dimensional digital models includes a total distance difference D (corresponding to the translation matrix T) and a total angle difference R (corresponding to the rotation matrix M). The total angle difference R may be calculated according to the following equation (1).

$$R = \cos^{-1} \frac{\mathrm{trace}(M)-1}{2} \qquad\qquad \text{equation (1)}.$$

Here, "trace" is a trace of the matrix.

After decomposing the total distance difference D and the total angle difference R in the reference coordinate system of the tooth to be estimated, the following components are obtained: a distance difference Dx in the mesiodistal direction, a distance difference Dy in the labiolingual direction, a distance difference Dz in the vertical direction, a torque difference Rx, an axial tilt difference Ry and a torsion difference Rz. Based on these components, dental professionals may more intuitively understand the medical significance of the pose difference of the tooth to be estimated between the analysis model and the reference model, so as to better guide subsequent orthodontic treatment.

In the above embodiment, the method of the present application is used to measure the pose difference of the tooth to be estimated between the analysis model and the reference model. However, it can be understood that the method of the present application is not limited to this application scenario. In fact, it can be used to measure the pose difference of the tooth to be estimated between any two different three-dimensional digital models representing the same dentition.

In orthodontic treatment using shell-style orthodontic appliances, in addition to the pose difference of the teeth to be estimated between the current tooth arrangement of patient and the target tooth arrangement of the current treatment step, the achievement rate of orthodontic treatment is also of great reference value for evaluating the effect of orthodontic treatment.

In an embodiment of the present application, a computer-implemented method for calculating the orthodontic treatment achievement rate of a tooth to be estimated includes the following steps.

In step 201, a pose difference of a tooth to be estimated between a current tooth arrangement and an original tooth arrangement of a patient is measured.

For ease of explanation, the pose difference of the tooth to be estimated between the current tooth arrangement and the original tooth arrangement of the patient is referred to as a first pose difference below.

First, a three-dimensional digital model of a dentition representing an initial tooth arrangement of a patient (i.e., a tooth arrangement of the patient before orthodontic treatment) and a three-dimensional digital model of a dentition representing a current tooth arrangement of the patient are obtained.

The three-dimensional digital model representing the initial tooth arrangement of the patient is used as the first three-dimensional digital model, and the three-dimensional digital model representing the current tooth arrangement of the patient is used as the second three-dimensional digital model. The method 100 is used to measure the components of the pose difference of the tooth to be estimated between the two tooth arrangements along the coordinate axes of the reference coordinate system of the tooth to be estimated, which are recorded as $D_{1x}$, $D_{1y}$, $D_{1z}$, $R_{1x}$, $R_{1y}$ and $R_{1z}$.

In step 203, a pose difference of the tooth to be estimated between a target tooth arrangement of a current treatment step and an original tooth arrangement is measured.

The pose difference of the tooth to be estimated between the target tooth arrangement of the current treatment step of the patient and the original tooth arrangement of the patient is referred to as a second pose difference.

In an embodiment, the current treatment step may be the last treatment step currently completed by the patient (i.e., the treatment step corresponding to the last shell-style orthodontic appliance currently completed for wear).

The three-dimensional digital model representing the initial tooth arrangement of the patient is used as the first three-dimensional digital model, and the three-dimensional digital model representing the target tooth arrangement of the current treatment step of the patient is used as the second three-dimensional digital model. The method 100 is used to measure the components of the pose difference of the tooth to be estimated between the two tooth arrangements along the coordinate axes of the reference coordinate system of the tooth to be estimated, which are recorded as $D_{2x}$, $D_{2y}$, $D_{2z}$, $R_{2x}$, $R_{2y}$ and $R_{2z}$.

In many cases, the pose differences of various teeth between the target tooth arrangement of the current treatment step and the original tooth arrangement are already known and do not need to be measured through registration.

In step 205, a component of the first pose difference is divided by a corresponding component of the second pose difference to obtain an orthodontic treatment achievement rate.

In an orthodontic treatment plan, orthodontics for a tooth may only involve a portion of the above six movement modes, for example, only mesial translation. For a tooth to be estimated, the achievement rate of the components corresponding to the movement modes involved in the orthodontic treatment plan is the most important indicator for evaluating the effect of orthodontic treatment. Therefore, in an embodiment, only the achievement rates of these components may be calculated.

In some cases, in actual orthodontic treatment, a tooth may undergo an undesirable movement, that is, a movement of the tooth is not included in the orthodontic design of the tooth in the orthodontic treatment plan. For example, the orthodontic treatment plan does not design a mesial translation for the tooth, but in actual orthodontic treatment, the tooth undergoes a mesial translation. In this case, the corresponding component of the second pose difference is zero. In an embodiment, the achievement rate may not be calculated for this component, and the corresponding component of the first pose difference may be used to evaluate the degree to which the actual orthodontic treatment deviates from the orthodontic treatment plan.

In an embodiment, if a component of the second pose difference is close to or equal to zero, a safety value may be added to the denominator when calculating the achievement rate to prevent it from being close to or equal to zero.

In an embodiment, if a component of the first pose difference is smaller than a preset threshold, it can be considered that the tooth has not moved in a corresponding direction.

In steps 201 and 203, the reference models are both three-dimensional digital models representing the original tooth arrangement of the dentition, based on which a reference coordinate system may be generated for each tooth for the consistency and simplification of calculations. That is, the occlusal surface, the dental arch curve and the corresponding points of various teeth on the dental arch curve may be determined based on it.

In the above embodiments, when calculating the orthodontic achievement rate, the three-dimensional digital model representing the target tooth arrangement of the currently completed treatment step of the patient is selected as a comparison object. In another embodiment, one of a series of successive three-dimensional digital models in the orthodontic treatment plan that is closest to the three-dimensional digital model representing the current tooth arrangement of the patient may also be selected as the comparison object. For example, in an embodiment, the one with the smallest residual of overall registration with the three-dimensional digital model representing the current tooth arrangement of the patient in the series of successive three-dimensional digital models may be selected as the closest one. In another embodiment, the one with the smallest step difference (calculated by dividing the component of the pose difference by the moving step length of each treatment step) with the three-dimensional digital model representing the current tooth arrangement of the patient in the series of successive three-dimensional digital models may also be selected as the closest one.

An aspect of the present application provides a computer-implemented method for measuring a tooth pose difference, including: obtaining a first three-dimensional digital model and a second three-dimensional digital model, which are two different three-dimensional digital models representing the same dentition; for each pair of corresponding teeth in the first and second three-dimensional digital models, performing coarse registration between the each pair of corresponding teeth based on local coordinate systems of the each pair of corresponding teeth; using an ICP method to finely register each pair of teeth that have been coarsely registered; selecting multiple reference points for each tooth in the first three-dimensional digital model to obtain a first reference point set; based on a result of the fine registration, projecting the reference points of each tooth in the first three-dimensional digital model to a corresponding tooth in the second three-dimensional digital model to obtain a second reference point set, here, the reference points in the first and second reference point sets form pairs of reference points; performing overall registration between the first and second three-dimensional digital models based on the first and second reference point sets; based on the first and second three-dimensional digital models after the overall registration, calculating a pose difference of a tooth to be estimated between the first and second three-dimensional digital models; establishing a reference coordinate system for the tooth to be estimated, wherein an extension direction of a z-axis of the reference coordinate system is consistent with a normal direction of an occlusal surface of the dentition, and an extension direction of an x-axis of the reference coordinate system is consistent with an extension direction of a tangent at a point on a dental arch curve of the dentition corresponding to the tooth to be estimated; and decomposing the pose difference in the reference coordinate system to obtain translation components along the x, y and z axes of the reference coordinate system and rotation components around the x, y and z axes of the reference coordinate system.

In some embodiments, the coarse registration is based on an SVD method.

In some embodiments, the coarse registration is performed by: for each pair of teeth, selecting a plurality of pairs of reference points in the local coordinate system of the two teeth, each pair of reference points having same coordinate values. The coarse registration of the pair of teeth is based on the plurality of pairs of reference points.

In some embodiments, a weight of a point pair on which the fine registration is based are assigned according to at least one of the following. (1) The weight is assigned according to a long axis coordinate of the local coordinate system, a point pair closer to an incisal edge or occlusal surface of the tooth has a higher weight, and a point pair closer to a gum line has a lower weight; (2) The weight is assigned according to the point pairs sorted by distance: in each iteration, the point pairs are sorted from large to small by distances, and a point pair with a larger distance has a lower weight. (3) The weight is assigned according to a distance threshold: in each iteration, based on that the distance of a point pair exceeds a preset distance threshold, a weight of the point pair is reduced.

In some embodiments, the computer-implemented method for measuring the tooth pose difference further includes: calculating a confidence level of the fine registration based on a proportion of point pairs that have completed the registration.

In some embodiments, for each pair of teeth, a first point in each point pair on which the fine registration is based is a vertex of a tooth of the first three-dimensional digital model in the pair of teeth, and a second point in the point pair is an intersection point of a ray casted along its normal direction from the first point with a facet of a tooth of the second three-dimensional digital model in the pair of teeth.

In some embodiments, the overall registration is based on the SVD method.

In some embodiments, in the overall registration, a weight of the reference point pairs on which the overall registration is based is assigned according to at least one of the following. (1) The weight is assigned according to a designed movement amount of each tooth in the treatment step corresponding to the second three-dimensional digital model, and the smaller the designed movement amount of the tooth corresponding to the reference point, the larger the weight. (2) The weight is assigned according to the confidence level of the fine registration, and the higher the confidence level of the fine registration of the tooth corresponding to the reference point, the larger the weight. (3) The weight is assigned according to the degree of difficulty in tooth movement, and the easier the movement of the tooth corresponding to the reference point is to achieve, the larger the weight. (4) The weight is assigned according to a position of the reference point on the tooth, and the closer the position to the crown, the larger the weight.

In some embodiments, the second three-dimensional digital model is a three-dimensional digital model representing the current state of the dentition, and the first three-dimensional digital model is one of a series of successive three-dimensional digital models in an orthodontic treatment plan using a shell-style orthodontic appliance, which represent target tooth arrangements of a series of successive treatment steps of the orthodontic treatment plan.

In some embodiments, the first and second three-dimensional digital models are obtained by two scans.

In some embodiments, the first three-dimensional digital model is one of the series of successive three-dimensional digital models that is closest to the second three-dimensional digital model.

Another aspect of the present application provides a computer-implemented method for calculating an achievement rate of orthodontic treatment, including: using the computer-implemented method for measuring the tooth pose difference to measure a pose difference of a tooth to be estimated between a three-dimensional digital model representing an initial tooth arrangement of a dentition and a three-dimensional digital model representing a current state of the dentition, denoted as a first pose difference, and decomposing the first pose difference in a reference coordinate system; measuring and obtaining a pose difference of the tooth to be estimated between the three-dimensional digital model representing the initial tooth arrangement of the dentition and a three-dimensional digital model representing the target tooth arrangement of the dentition, denoted as a second pose difference, and decomposing the second pose difference in the reference coordinate system; and obtaining an achievement rate of the tooth to be estimated in a corresponding movement direction by dividing a component of the first pose difference by a corresponding component of the second pose difference.

In some embodiments, the target tooth arrangement is a target tooth arrangement for a current treatment step in an orthodontic treatment plan using a shell-style orthodontic appliance.

Another aspect of the present application provides a computer system for measuring a tooth pose difference, which includes a storage device and a processor, wherein the storage device stores a computer program for measuring tooth pose difference, and when the computer program is run by the processor, it executes the computer-implemented method for measuring a tooth pose difference as described in claim 1.

The application proposes a method for evaluating the difference between the actual tooth status and the planned solution, including the estimation of single tooth, jaw position changes, step number lags, etc., to give efficient, stable, high-precision, three-dimensional results.

In some embodiments, the estimation information of the at least one tooth to be estimated includes at least one of the following: absolute change amount of a single tooth, relative change amount of a single tooth, jaw position deviation information of at least one tooth to be estimated, and step number deviation information.

In some embodiments, during the tooth deviation estimation process, the input data may include at least one of the following.

Original tooth model: a digital model of one or more teeth of a patient before the start of treatment (or at an earlier point in the treatment) may include all teeth, which is called model C and may be a second three-dimensional digital model, that is, the second three-dimensional digital model may be a digital model of one or more teeth of the patient collected and obtained before the start of treatment.

Tooth model corresponding to the treatment plan: based on model A, a treatment plan is designed to be completed step-by step, which is called model $B_1$,..., $B_n$ according to steps 1 to n. The tooth model may be the first three-dimensional digital model, that is, a digital model of the design plan corresponding to the second three-dimensional digital model, and the digital model may be located in the same coordinate system as model A.

New tooth model: during treatment (at a later point in the treatment), a digital model of one or more teeth of the patient may include all teeth and is called model A, which may be a second three-dimensional digital model, that is, the first three-dimensional digital model may be a digital model of one or more teeth of the patient collected and obtained after the start of treatment.

A registration result of at least one tooth: the registration of model C (or at least one of models $B_1$, ..., $B_n$) with model A. The registration method may refer to the above embodiment and will not be repeated here. It should be noted that all the above models are located in the same coordinate system after registration.

The present application provides a method for estimating the deviation of a single tooth.

Determining the teeth to be estimated to obtain the first and second three-dimensional digital models may include: obtaining a collected digital model of the teeth to be estimated, and obtaining step information to be estimated. Based on the step information to be estimated, a three-dimensional digital model of a design solution corresponding to the step information (i.e., the second three-dimensional digital model to be estimated) may be obtained.

The method for determining the step information may refer to the above embodiment, or may be determined independently, which is not limited here.

For example, determining the step information of the first three-dimensional digital model to be estimated may be based on at least one of the following.

For example, the step information of the first three-dimensional digital model to be estimated may be determined according to specified step information.

For example, the step information of the first three-dimensional digital model to be estimated may be determined according to the best match step information included in registration results of multiple teeth.

For example, a difference between model $B_1$, ..., $B_n$ and model A may be calculated, and a step corresponding to a model B with the smallest difference is selected as the step information of the first three-dimensional digital model to be estimated. The specific degree of difference may be determined based on the registration result of each tooth in teeth to be estimated, and a method for determining the degree of difference is not limited here.

Taking the comparison between model A and model B as an example, the deviation of the teeth is estimated, and the estimation information may include the estimation result of a single tooth, and the estimation result may include at least one of the following: the absolute change amount of a single tooth, and the relative change amount of a single tooth.

Optionally, the method includes: determining a reference system.

For example, in order to make the estimation of tooth movement closer to clinical cognition, a point on the dental arch corresponding to the tooth is usually used to establish a space coordinate system as a reference system for tooth movement.

For example, based on model A (or model C or a model $B_k$), a normal direction of a jaw plane, midline, and a dental arch curve may be calculated (the specific method is not expanded here)

For example, for a certain tooth to be estimated, a corresponding point on the dental arch curve may be found; for example, a point closest to a tooth in model C, or a point closest to a tooth in model $B_k$.

For example, a coordinate system can be established with the corresponding point as an origin, the z-axis is the normal direction of the jaw plane, the x-axis is a direction tangent to the dental arch and perpendicular to the z-axis, and the y-axis is a direction perpendicular to both the z-axis and the x-axis.

Optionally, the estimation result may include an absolute change amount.

For example, a certain tooth that needs to be estimated may be determined, and a first three-dimensional digital model, i.e., model A, may be obtained.

For example, a step number k may be determined, and a first three-dimensional digital model, i.e., model $B_k$, may be obtained.

For example, tooth models of the tooth to be estimated may be found in model A and model $B_k$, respectively, and single tooth registration may be performed on the two tooth models (the specific method may refer to the above implementation).

For example, the translation amount in the above registration result can be used, and the length of the translation amount is a total distance of tooth movement; the translation amount is projected to the reference system determined above, where the x-axis component represents the mesiodistal direction, the y-axis component represents the labiolingual direction, and the z-axis component represents the vertical direction.

For example, the rotation amount in the above registration result is represented by the rotation matrix M, and the total rotation angle of the tooth is $\cos^{-1} \frac{\text{trace}(M)-1}{2}$ .

This rotation amount is decomposed into the reference system determined above, where the rotation angle around the x-axis is the torque, the rotation angle around the y-axis is the axis tilt, and the rotation angle around the z-axis is the torsion.

For example, an output estimation result may include at least one of the following: a total tooth movement distance d, a translation amount dx in a mesiodistal direction, a translation amount dy in a labiolingual direction, a translation amount dz in a vertical direction.

Optionally, the difference between the actual state of the tooth and the designed state of the tooth may be expressed based on at least one of the above 8 values, and the progress and effect of the treatment can be estimated in combination with clinical data and experience.

Optionally, the estimation result may include a relative change amount.

For example, a certain tooth to be estimated may be determined and step information may be determined as described above, and will not be described in detail here.

For example, the tooth models of the tooth to be estimated may found in model $B_k$ and model A, respectively, and single tooth registration is performed on the two models (the specific method may be found above)

For example, the tooth models to be estimated may be found in model C and model A, respectively, and single tooth registration is perform on the two models (the specific method may refer to the above)

For example, based on a registration result of model $B_k$ and model A, the designed movement result of the tooth to be estimated may be obtained, which are recorded as d, dx, dy, dz, R, Rx, Ry, and Rz; these values represent the designed movement amount of the tooth.

For example, based on a registration result of model C and model A, the actual movement result of the tooth to be estimated may be obtained, which are recorded as d', dx', dy', dz', R', Rx', Ry', and Rz'; these values represent the actual movement amount of the tooth.

Optionally, the relative change amount may be a ratio of the actual movement amount to the designed movement amount.

Optionally, in order to reduce misjudgment caused by errors in each link, when the designed movement amount is close to zero, a safety value may be added to ensure that the denominator in the proportional calculation is not close to or equal to zero; when the actual movement amount is less than a certain threshold, it can be set to zero, that is, it is considered that the tooth has not moved in this direction.

For example, for d'/d and R'/R, the numerator and denominator are both non-negative, so the closer the ratio is to 1, the closer the actual situation is to the design; for the other 6 ratios dx'/dx, dy'/dy, dz'/dz, Rx'/Rx, Ry'/Ry, and Rz'/Rz, the numerator and denominator all may be positive or negative, and the closer the ratio is to 1, the closer the actual situation is to the design. If the ratio is between 0 and 1, it means that the tooth moves in the designed direction but the movement amount of the tooth is insufficient. If the ratio is less than 0, it means that the tooth moves in an opposite direction to the design. If the ratio is greater than 1, it means that the tooth moves in the designed direction and the movement amount of the tooth exceeds the design.

As shown in FIG. 4A, single tooth estimation (relative change amount) is shown. The red model on the left represents the initial position of the tooth, the orange model represents the designed position of the tooth, and the red arrow represents the tooth movement between the red model and the orange model (only the highest point translation is shown); the red model on the right represents the initial position of the tooth, the orange model represents the actual position of the tooth, and the blue arrow represents the tooth movement between the two model (only the highest point translation is shown); by comparing the red and blue arrows, the tooth movement estimation based on the relative change amount is obtained.

In medicine, the upper jaw of the human is considered to be fixed, and jaw position change refers to the change in the jaw position of the lower jaw.

Orthodontic treatment may involve jaw position changes, such as mandibular advancement, mandibular deviation correction, and changes in dental arch curves caused by tooth position changes. In order to evaluate the effect of orthodontic treatment, it is necessary to measure jaw position changes.

The present application provides a method for evaluating jaw position changes. In this embodiment, estimation information may include: jaw position deviation information.

Optionally, at least one of the following may be included: determining step information and a reference system.

Taking the comparison between model A and model B as an example, the comparison between model C and model B, and the comparison between model A and model C may also refer to this method, which will not be repeated here.

For example, the method for determining the step information is as follows: the step number of model $A^{up}$ (the superscript represents all teeth in a single jaw in the model) is determined to obtain $B_k^{up}$, mandibular model $B_k^{down}$ and model $A^{down}$ with the same step number is select.

Optionally, a reference system is determined.

For example, based on model A, the normal direction of the jaw plane and midline are calculated. The normal direction of the jaw plane is the z-axis, indicating the up-down direction; the midline is the y-axis, indicating the front-back direction; and the x-axis is the direction perpendicular to both the y-axis and the z-axis, indicating the left-right direction.

Optionally, the jaw position change may be calculated in the reference system determined above.

In some embodiments, the estimation information may include jaw position deviation information, and the jaw position deviation information includes: translation and rotation amounts. The jaw position deviation information of at least one tooth to be estimated is determined according to the following manner:

obtaining first reference jaw position deviation information according to a first three-dimensional digital model of a first jaw position of a patient and a first three-dimensional digital model of a second jaw position of the patient;

obtaining second reference jaw position deviation information according to a second three-dimensional digital model of the first jaw position of the patient and a second three-dimensional digital model of the second jaw position of the patient;

obtaining a registration result of at least one tooth according to the first three-dimensional digital model of the first jaw position of the patient and the second three-dimensional digital model of the first jaw position of the patient, and determining translation information and rotation information of the at least one tooth; and

obtaining the jaw position deviation information of the at least one tooth to be estimated according to the translation information and rotation information of at least one tooth, and the first and second reference jaw position deviation information.

The first jaw position may be the upper jaw or the lower jaw, and the second jaw position is the jaw position corresponding to the first jaw position.

For example, a relative position relationship between models $A^{down}$ and $A^{up}$ may be calculated to obtain translation amount $t_1$ and rotation matrix $M_1$.

For example, a relative position relationship between models $B_k^{down}$ and $B_k^{up}$ may be calculated to obtain translation amount $t_2$ and rotation matrix $M_2$.

For example, the registration problem of multiple teeth may be solved based on models $B_k^{up}$ and $A^{up}$, to obtain translation amount $t_3$ and rotation matrix $M_3$.

For example, it can be represented by a translation amount and a rotation amount.

Optionally, the total translation amounts $t = t_2 + t_3 - t_1$ and the total rotation matrix $M = M_1^T M_3 M_2$ may be calculated.

The above results are decomposed into the reference system to obtain the translation components along the three coordinate axes, which respectively represent the translation change amount of the mandibular jaw position in the left-right, front-back, and up-down directions (a comparison between the actual state and the designed state).

The above results are decomposed into the reference system to obtain the rotation angles around the three coordinate axes, which respectively represent the angular variation of the mandibular jaw position in terms of pitch, roll, and yaw (a comparison between the actual state and the designed state).

Optionally, it can be indicated by a position of a key tooth.

In some embodiments, the jaw position deviation information includes deviation information of the position of the key tooth, and the deviation information of the position of the key tooth includes: translation change amount between actual state and design state of key tooth.

For example, a certain tooth in $A^{down}$ may be selected, such as teeth numbered 31, 41, 33, 43, 36, 46, etc. are selected.

For example, for each tooth (whose center is the three-dimensional vector v), a three-dimensional vector $Mv+t$ may be calculated, and three components of the three-dimensional vector respectively represent the translation change amount of the tooth in the left-right, front-back, and up-down directions (a comparison between the actual state and the designed state).

Optionally, it may be indicated by clinical indicators.

Specifically, this may be performed by: obtaining reference points for the clinical indicators;

determining jaw position information of a clinical indicator of the first three-dimensional digital model according to a reference point of the clinical indicator corresponding to the first three-dimensional digital model;

determining jaw position reference information of a clinical indicator of the second three-dimensional digital model according to a reference point of a clinical indicator corresponding to the second three-dimensional digital model; and

determining the jaw position deviation information according to the jaw position information of the clinical indicator and the jaw position reference information of the clinical indicator.

Taking anterior overjet as an example, the calculation methods of other indicators are similar, such as anterior overbite, posterior overjet, posterior overbite, sagittal relationship of molars, a molar occlusal depth, etc.

For example, the reference points of the anterior overjet may be calculated in the models $A^{down}$ and $A^{up}$, respectively, and projected to a certain reference system to calculate the anterior overjet in the model A.

For example, the reference points of the anterior overjet may be calculated in the models $B_k^{down}$ and $B_k^{up}$, respectively (in order to reduce the error in the reference point calculation, a single tooth registration and projection method may be used), and projected to a certain reference system to calculate the anterior overjet in the model $B_k$.

As shown in FIG. 4B, the estimation of jaw position changes. The orange rectangle on the left represents the designed upper and lower jaw models (the relative position relationship represents the jaw position), and the green rectangle in the middle represents the actual upper and lower jaw models (the relative position relationship represents the jaw position); the right side shows a result after maxillary registration (the orange maxillary and green maxillary completely overlap), at this time there is a significant difference in the mandible, and the translation and rotation amounts may be calculated.

The present application provides a method for estimating deviation of the number of steps. In this embodiment, the estimation information may include: step deviation results, which may include:

obtaining change amount of teeth in a plurality of digital models corresponding to a plurality of steps;

according to a distance between estimated step information and actual step information, determining step deviation information of the teeth.

The method for determining the step deviation information for a single tooth may include at least one of the following.

Optionally, for a tooth model corresponding to one step, a translation amount dx in a mesiodistal direction, a translation amount dy in a labiolingual direction, a translation amount dz in a vertical direction, torque Rx, axial tilt Ry, and torsion Rz of the single tooth are calculated.

For example, the above process may be repeated in all steps 1, ..., n to obtain dx, dy, dz, Rx, Ry, and Rz in each step.

For example, for dx, a certain step range may be searched, and the one with the smallest absolute value of dx in the certain step range may be found to obtain its corresponding step number 1, which means that this step number is closest to the current actual tooth state.

For example, if the step number corresponding to an orthodontic appliance currently worn is k, then $lag_{dx} = k-1$, indicating the distance of the actual tooth state from the step number of the current orthodontic appliance.

For example, the above operation may be repeated for dy, dz, Rx, Ry, and Rz to obtain $lag_{dy}$, $lag_{dz}$, $lag_{Rx}$, $lag_{Ry}$, $lag_{Rz}$.

For example, for a single tooth, $lag = max\{lag_{dx}, lag_{dy}, lag_{dz}, lag_{Rx}, lag_{Ry}, lag_{Rz}\}$.

Overall estimation of a single tooth: "lag" represents the number of steps by which the tooth lags behind the design state; if the "lag" is equal to zero, it means that the tooth moves basically according to the design; if the "lag" is greater than zero, it means that the tooth movement lags behind, and the larger the "lag", the greater the lag; if "lag" is less than zero, it means that the tooth moves ahead of schedule.

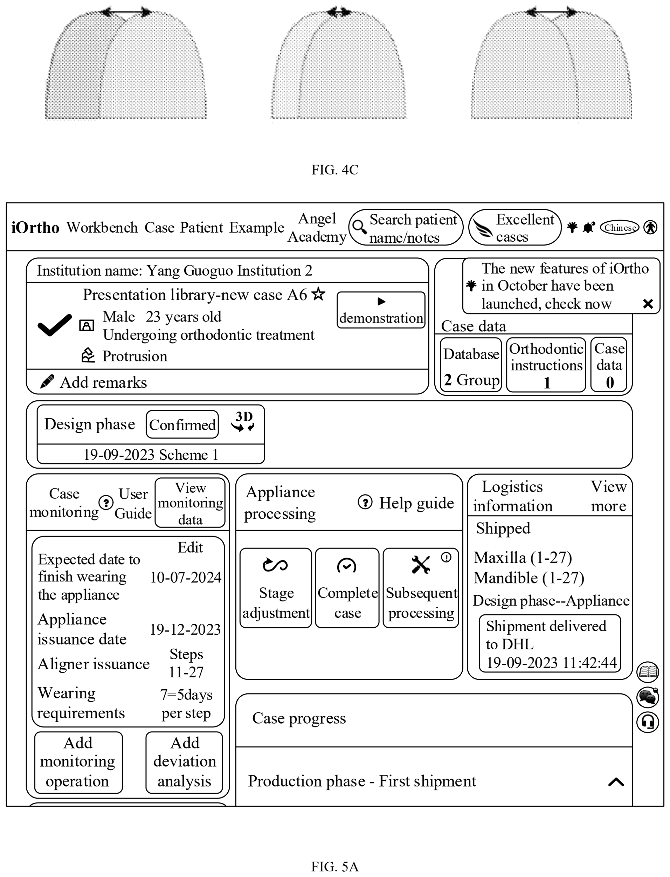

As shown in FIG. 4C, in the three pictures, green represents an actual position of the tooth, and red, orange, and blue represent the designed positions of the tooth under three different steps, respectively. After registration, amounts of tooth movement corresponding to the three steps are calculated respectively, the amounts of tooth movement represent by black arrows (only the translation amount of the highest point is shown). The amount of movement corresponding to orange is the smallest, so it is judged that this step is closest to the current actual tooth state.

In the embodiments of the present application, a method for calculating and estimating tooth movement is provided by combining information such as the step status of teeth in the treatment plan and the current actual status of teeth. A method for calculating the absolute change amount and relative change amount of a single tooth. A method for calculating the relative jaw position change of another jaw with a jaw as a reference. Three methods for displaying jaw position changes. A method for estimating the number of steps by which tooth movement lag (deviation) relative to the treatment plan.

Through the above methods, a plurality of methods for estimating tooth movement are provided, covering aspects such as a single tooth, a jaw position, a lagging condition, etc. These help timely discover and correct problems that occur during treatment. Estimation information based on three-dimensional data is given, replacing the three-dimensional measurement that is difficult to achieve in manual estimation, providing richer information and results that are closer to reality. All steps can be automated without human intervention, which improves efficiency, reduces subjective factors, and improves the accuracy of calculation results.

On the one hand, the present application provides a computer-implemented method for measuring a jaw position difference, which is used to measure the jaw position difference between two different three-dimensional digital models representing the dento-jaw of the same patient.

On the other hand, the present application provides a computer system for measuring a jaw position difference, which includes a storage device and a processor. The storage device stores a computer program for measuring the jaw position difference. When the computer program is run by the processor, the processor will execute the method for measuring the jaw position difference.

The computer-implemented method for measuring the jaw position difference in an embodiment of the present application includes the following steps.

In step 101, first and second three-dimensional digital models are obtained.

The first and second three-dimensional digital models are two different three-dimensional digital models representing maxillary and mandibular dentitions of the same patient, that is, each of the first and second three-dimensional digital models includes a maxillary portion an a mandibular portion..

In an embodiment, the first three-dimensional digital model is a reference model, which is used as a reference for comparison, and the second three-dimensional digital model is an analysis model, which is used as a comparison object. The method of the present application may be used to measure the jaw position difference between the analysis model and the reference model.

An application scenario of the method for measuring the jaw position of the present application is that during orthodontic treatment using a shell-style orthodontic appliance, a three-dimensional digital model (i.e., the analysis model) representing the current dento-jaw of the patient is compared with a three-dimensional digital model (i.e., the reference model) that is selected from multiple successive three-dimensional digital models in an orthodontic treatment plan using a shell-style orthodontic appliance, and the jaw position difference between the analysis model and the reference model is measured to evaluate the treatment effect based on the measurement results.

Another application scenario of the method for measuring the tooth pose difference of the present application is that after the orthodontic treatment is completed, the three-dimensional digital model (i.e., the analysis model) representing the current dento-jaw of the patient is compared with a three-dimensional digital model (i.e., the reference model) representing the jaw in the target state (i.e., the dento-jaw state desired to be achieved by orthodontic treatment), and the jaw position difference between the analysis model and the reference model is measured to judge the effect of the orthodontic treatment based on the measurement results.

It can be understood that the method for measuring jaw position difference of the present application is not limited to the above two application scenarios, and it is applicable to any application scenario of measuring the jaw position difference between two different three-dimensional digital models representing the dento-jaw of the same patient.

The following uses the first application scenario as an example to describe in detail the method for measuring jaw position difference of the present application.

The three-dimensional digital model representing the dento-jaw of the patient is usually obtained by scanning, for example, intraoral scanning, or scanning a physical model (such as a plaster model) or an impression of the dento-jaw of the patient. The maxillary and mandibular portions of the three-dimensional digital model representing maxillary and mandibular dentition of the patient obtained by scanning are usually in an occluded state, that is, a relative position relationship between the maxillary dentition and the mandibular dentition is known. Therefore, if the maxillary portions of two different three-dimensional digital models representing the dento-jaw of the same patient are aligned, a pose difference between the mandibular portions of the two different three-dimensional digital models may be measured. The pose includes position and posture, and the pose difference may also be referred to as position difference in this application). It can be understood that in addition to the occluded state, the maxillary and mandibular portions of the three-dimensional digital model representing maxillary and mandibular dentitions of the patient obtained by scanning may also be in other states, for example, the maxillary and mandibular portions are relatively translated a distance or rotated an angle from the occluded state, as long as the maxilla and mandible of the first three-dimensional digital model and the maxilla and mandible of the second three-dimensional digital model are in the same state.

As is known to those skilled in the art, orthodontic treatment of a dentition (maxillary or mandibular dentition) using shell-style orthodontic appliances usually requires dozens of successive shell-style orthodontic appliances, each shell-style orthodontic appliance corresponding to a treatment step, which is used to reposition the dentition from the tooth arrangement achieved in the previous treatment step to the target tooth arrangement of the current treatment step.

In orthodontic treatment, usually, the maxillary dentition and the mandibular dentition need to be treated simultaneously, so there are dozens of successive shell-style orthodontic appliances for each dentition. Depending on the specific conditions of the dentitions, the total number of treatment steps for the maxillary dentition and the mandibular dentition (i.e., the total number of shell-style orthodontic appliances) may be different. In the orthodontic treatment plan, the treatment steps of the maxillary dentition and the mandibular dentition are paired, that is, a plan is defined in which the treatment steps of the maxillary dentition and the mandibular dentition are performed simultaneously.

For each of the maxillary dentition and the mandibular dentition, the orthodontic treatment plan includes a series of successive three-dimensional digital models, which respectively represent target tooth arrangements of a series of successive treatment steps of a corresponding dentition. In an embodiment, a series of successive three-dimensional digital models of a dentition may be obtained as follows. First, before orthodontic treatment, a three-dimensional digital model representing an original tooth arrangement of the dentition of the patient is obtained by scanning. Next, the three-dimensional digital model representing the original tooth arrangement of the dentition is operated to obtain a three-dimensional digital model representing a target tooth arrangement of the dentition during orthodontic treatment. Then, based on the three-dimensional digital model representing the original tooth arrangement and the three-dimensional digital model representing the target tooth arrangement for orthodontic treatment, three-dimensional digital models representing a series of successive tooth arrangements between them is generated.

In an orthodontic treatment plan, each treatment step corresponds to a three-dimensional digital model representing a target tooth arrangement of the maxillary dentition and a three-dimensional digital model representing a target tooth arrangement of the mandibular dentition in the treatment step. In an embodiment, the first three-dimensional digital model may be a three-dimensional digital model (including the maxillary portion and the mandibular portion) representing the target tooth arrangement of one treatment step in the orthodontic treatment plan.

In an embodiment, a three-dimensional digital model representing a dento-jaw of the patient in the current state may be obtained by scanning. Since the first and second three-dimensional digital models are obtained by two scans, the first and second three-dimensional digital models may be in different world coordinate systems. In addition, the morphology of teeth of the patient may change during orthodontic treatment (for example, due to factors such as wear or growth). Therefore, the first and second three-dimensional digital models need to be registered before comparing them.

Due to the need for subsequent processing, the first and second three-dimensional digital models three-dimensional digital models that have been segmented, that is, the teeth thereof are independent of each other.

In step 103, performing coarse registration between teeth corresponding to maxillary portions of the first and second three-dimensional digital models.

Since a relative position relationship between the maxilla and mandible of the first and second three-dimensional digital models is known, the jaw position difference between the first and second three-dimensional digital models can be calculated as long as the maxillary portions of the two are registered.

In an embodiment, the teeth in the first and second three-dimensional digital models may be numbered in a predetermined manner, and the teeth in the maxillary portions of the first and second three-dimensional digital models may be paired in pairs according to the tooth numbers, i.e., the same tooth may be found in the maxillary portions of the first and second three-dimensional digital models.

In order to facilitate calculation, for a three-dimensional digital model of a dento-jaw, in addition to setting a world coordinate system for it, a local coordinate system is also set for the three-dimensional digital model of each tooth.

Since setting the local coordinate system using current technology (e.g., a local coordinate system setting method based on deep learning) may achieve extremely high accuracy and consistency, in an embodiment, two three-dimensional digital models of the same tooth may be coarsely registered based on the local coordinate system.

In an embodiment, for the three-dimensional digital model of the same tooth in the maxillary portions of the first and second three-dimensional digital models, at least three points in its local coordinate system may be selected as reference points, and the two three-dimensional digital models of the tooth may be coarsely registered based on these reference points. For example, the four points (0,0,0), (1,0,0), (0,1,0) and (0,0,1) may be used as the reference points. It is understood that the selection of reference points is not limited to this example, as long as they are not on the same straight line.

In some cases, there may be differences between the three-dimensional digital models of the same tooth in the maxillary portions of the first and second three-dimensional digital models. For example, tooth wear, addition/removal of attachments, or changes in the gum line (for example, which may be caused by the growth of erupted teeth, vertical movement or tilting of teeth, etc.) may cause a difference between the three-dimensional digital models of the same tooth scanned at different times.

If there are large differences between the two three-dimensional digital models of the same tooth, for example, a morphological difference between the three-dimensional digital models obtained by scanning at different time points during the tooth eruption process may be large, and the two three-dimensional digital models may not be well coarsely registered based on the local coordinate system. In this case, feature points may be used as reference points for registration, such as buccal cusp points, FA points, and adjacent points. At present, there are many methods for identifying feature points on three-dimensional digital models of teeth, such as a feature point recognition method based on deep learning. The recognition of feature points will not be described in detail here.

In an embodiment, two three-dimensional digital models of the same tooth may be measured, for example, the mesiodistal width and crown height of the tooth, and the measured difference and a preset threshold may be compared to determine whether there is a large difference between the two three-dimensional digital models of the same tooth. If there is a large difference, the feature points are used as reference points for coarse registration, otherwise, coarse registration is performed based on the local coordinate system.

In an embodiment, a Singular Value Decomposition (SVD) method can be used to coarsely register the teeth corresponding to the maxillary portions of the first and second three-dimensional digital in pairs models based on the reference points.

In step 105, based on a coarse registration result, fine registration is performed on teeth corresponding to maxillary portions of the first and second three-dimensional digital models.

After the coarse registration, the teeth of the maxillary portion of the first three-dimensional digital model are coarsely registered with the corresponding teeth of the maxillary portion of the second three-dimensional digital model. On this basis, these teeth can be finely registered in pairs.

In an embodiment, an iterative closest point algorithm (hereinafter referred to as ICP algorithm) may be used to finely register two three-dimensional digital models of the same tooth.

In an embodiment, two three-dimensional digital models of the same tooth may be finely registered based on a vertex-to-surface approach. For ease of description, the two three-dimensional digital models of the same tooth are hereinafter referred to as model A and model B, respectively.

In an embodiment, the point pairs for the fine registration may be determined according to the following method. Some vertices sampled or all vertices selected from model A are taken as a first point set for the fine registration. For each point in the first point set, a ray is cast from the point along a normal direction, an intersection point of the ray with model B (i.e., an intersection with a surface of model B) is obtained, and a starting point of the ray and the intersection point are taken as a point pair.

Since the relative position relationship between models A and B is unknown, the intersection point of a unidirectional ray with model B is not necessarily a valid intersection point. Therefore, rays may be cast from a vertex on model A along the normal direction in two opposite directions, or a straight line may be drawn passing through the vertex on model A. in this way, two intersection points with model B may be obtained, and the closer intersection point is selected.

In addition, model B may lack a portion which is in model A. Therefore, a threshold may be set. If the distance between a vertex and each corresponding intersection point is greater than the threshold, it is considered that the rays from the vertex along the normal direction have no valid intersection point with model B.

In yet another embodiment, two three-dimensional digital models of the same tooth may be finely registered based on a vertex-to-vertex approach.

In an embodiment, the point pairs for the fine registration may be determined according to the following method: some vertices sampled or all vertices selected from model A are taken as a first point set for registration. For each point in the first point set, the vertex on model B closest to the point is found, and the two vertices are taken as a point pair.

In an embodiment, a distance threshold may be set. During the iteration process, if the distance of a point pair is less than the distance threshold, the point pair is considered to have completed the registration. In an embodiment, the distance threshold may be determined based on the accuracy of a scanning device used to generate the first and/or second three-dimensional digital models. For example, if the accuracy of the scanning device used is 0.1 mm, the distance threshold may be set to 0.1 mm, or 0.08 mm, or 0.12 mm, etc.

In an embodiment, a proportion threshold may be set. If the proportion of the point pairs that have completed the registration is greater than the proportion threshold, it is considered that the registration of the models A and B is completed.

In an embodiment, the following conditions may be set. If any one of these conditions is met, the iteration is stopped: (1) the proportion of point pairs that have completed the registration is greater than the proportion threshold; (2) the number of iterations exceeds a preset iteration number threshold; and (3) the difference between the pose after the current iteration and the pose after the previous iteration is less than a preset pose difference threshold (a comprehensive estimation based on translation and rotation amounts).

Although the models A and B correspond to the same tooth, as mentioned above, due to wear, addition/removal of accessories, and changes in the gum line, the models A and B may not completely overlap. Therefore, the influence of these factors needs to be minimized as much as possible during the registration process.

In an embodiment, at least one of the following methods may be used to assign a weight to a point pair on which the fine registration is based, so as to minimize the influence of the above factors on the fine registration.

(1) A weight is assigned according to a long axis coordinate of the local coordinate system. A point pair close to the incisal edge or occlusal surface of the tooth has a higher weight, while a point pair close to a gum line has a lower weight, so as to minimize the interference caused by the change of the gum line.

(2) A weight is assigned according to point pairs sorted by distance. In each iteration, point pairs are sorted from large to small by distance, and a point pair with a larger distance has a lower weight, so as to minimize interference caused by morphological differences.

(3) A weight is assigned according to a distance threshold. The distance threshold may be set in advance. For example, the distance threshold may be set based on the scanning accuracy. In each iteration, if the distance of a point pair exceeds the distance threshold, it is considered that the distance of the point pair is caused by morphological differences, and a weight of the point pair is reduced accordingly. The weight of the point pair may even be reduced to zero, i.e., the point pair does not participate in this iteration.

When the iteration of fine registration stops, the following results are output.

(1) A rigid transformation (translation and rotation in three-dimensional space) between the models A and B.
(2) A confidence level: the percentage of point pairs that have completed registration. The higher the proportion, the higher the confidence level, indicating that the registration result is more reliable and can be used as a reference for subsequent processing.
(3) An anomalous point: a point pair(s) that has/have not completed registration when the iteration stops. For example, the distance(s) of the point pair(s) may be compared with the above-mentioned distance threshold, and the point pair(s) with distance(s) greater than the distance threshold is/are regarded as the morphological difference(s) between the models A and B.

In step 107, performing overall registration between the maxillary portions of the first and second three-dimensional digital models based on a result of the fine registration, and calculating and obtaining a jaw position difference between the first and second three-dimensional digital models based on a result of the overall registration.

In an embodiment, a reference point of each tooth in the maxillary portion of the first three-dimensional digital model may be projected to a corresponding tooth in the second three-dimensional digital model according to the result of the fine registration (the coordinates of the reference point of the second three-dimensional digital model are consistent with the coordinates of the corresponding reference point of the first three-dimensional digital model), and based on the two reference point set, the maxillary portions of the first and second three-dimensional digital models are registered as a whole.

In an embodiment, a reference point of each tooth in the maxillary portion of the first three-dimensional digital model may use a reference point selected by the coarse registration. It is understood that a reference point may also be re-selected for each tooth in the maxillary portion of the first three-dimensional digital model, and the same method as in the coarse registration may be used to re-select the reference point.

Since the tooth arrangement actually achieved when completing a treatment step is likely to be different from the target tooth arrangement of the treatment step, in this case, the entire maxillary portions of the first and second three-dimensional digital models cannot completely overlap, so it is desirable to focus on the registration of stationary teeth during registration. Therefore, when performing overall registration, on the one hand, a weight may be assigned according to a designed movement amount of each tooth in the maxillary portion of the first three-dimensional digital model at the corresponding treatment step, and the smaller the designed movement amount, the greater the weight.

On the other hand, a weight may be assigned according to a confidence level of the registration result of a single tooth, and the higher the confidence level, the greater the weight.

On the other hand, a weight may also be assigned according to the degree of difficulty in tooth movement. For movements that are relatively easy to achieve, after completing a treatment step, the pose of the tooth is closer to the target pose of the treatment step, so a larger weight can be assigned to the tooth.

In yet another aspect, a weight may be assigned according to a position of a reference point on the tooth, the closer the reference point is to the crown, the greater the weight.

At least one of the above methods may be used to assign weights to the reference point pairs. After the weights are assigned to the reference points, overall registration may be performed based on these reference points.

In an embodiment, the SVD method may be used to perform overall registration based on the reference points. The obtained results include a rigid transformation and confidence level.

In an embodiment, the confidence level is based on the residual after registration, and the larger the residual, the lower the confidence level.

In yet another embodiment, a normal distribution transformation method (NDT for short) may be used to perform overall registration based on the reference points.

In an embodiment, the sum of the squares of the Mahalanobis distances of the reference points after registration may be used as a confidence level indicator. The larger the value of the sum of the squares, the lower the confidence level.

In yet another embodiment, a weight may be assigned to each tooth, and a weighted average transformation may be calculated and obtained based on the rigid transformation obtained by the fine registration, and the weighted average transformation may be used as the overall registration result of the first and second three-dimensional digital models.

In an embodiment, a weight of each tooth may be set according to at least one of the following: (1) the weight is assigned according to a designed movement amount of the tooth (for example, the designed movement amount of a corresponding treatment step, or the total designed movement amount from the original tooth arrangement to a next corresponding treatment step), and the smaller the designed movement amount, the greater the weight; (2) the weight is assigned according to a confidence level of a registration result of a single tooth, and the higher the confidence level, the greater the weight; (3) the weight is assigned according to the degree of difficulty in tooth movement (for example, the movement of a corresponding treatment step, or the movement from the original tooth arrangement to a next corresponding treatment step), and the easier the movement is to achieve, the greater the weight.

For each tooth, a difference between the rigid transformation of the fine registration and the weighted average transformation is calculated, and all differences are summed (taking the absolute value to keep the value non-negative) to obtain the residual of the registration. The residual of the registration may be used as an indicator of the confidence level. The higher the residual, the lower the confidence level.

After the maxillary portions of the first and second three-dimensional digital models are overall registered, it can be considered that the maxillary portions of the first and second three-dimensional digital models have been aligned, on this basis, the pose difference of the mandibular portions between the first and second three-dimensional digital models can be calculated.

The relative positional relationship between the maxillary portion and the mandibular portion of the first and second three-dimensional digital models is known. Assume that the relative positional relationship between the maxillary portion and the mandibular portion in the first three-dimensional digital model is $T_1$ (translation matrix, representing the translation component) and $M_1$ (rotation matrix, representing the rotation component), and the relative positional relationship between the maxillary portion and the mandibular portion of the second three-dimensional digital model is $T_2$ (translation matrix, representing the translation component) and $M_2$ (rotation matrix, representing the rotation component). The result of the overall registration is $T_3$ (translation matrix, representing the translation component) and $M_3$ (rotation matrix, representing the rotation component). The translation component $T$ and the rotation component $M$ of the pose difference of the mandibular between the first and second three-dimensional digital models can be calculated according to the following equations (1) and (2), respectively:

$$T = T_2 + T_3 - T_1 \qquad \text{equation (1)};$$

$$M = M_1^{trans} \times M_3 \times M_2 \qquad \text{equation (2).}$$

Here, trans represents the transpose of the matrix.

In an embodiment, the relative position relationship between the maxillary dentition and mandibular dentition may be calculated according to the following method.

First, a normal direction of a jaw plane and midline are calculated based on a three-dimensional digital model of a maxillary dentition. The normal direction of the jaw plane is used as the z-axis of its reference coordinate system, and the midline is used as the y-axis of its reference coordinate system. The x-axis of its reference coordinate system is perpendicular to the z-axis and y-axis. The average value of the centers of gravity of teeth is calculated as an origin of its reference coordinate system. The same operation is performed on the three-dimensional digital model of the mandibular dentition to calculate and obtain its reference coordinate system.

Then, a relative position relationship between the reference coordinate system of the three-dimensional digital model of the maxillary dentition and the reference coordinate system of the three-dimensional digital model of the mandibular dentition is calculated. In an embodiment, the relative position relationship may be expressed by a translation matrix and a rotation matrix.

It can be understood that the calculation of the reference coordinate system is not limited to the above method. For example, the origin of the reference coordinate system may also be a midpoint of a line connecting the centers of gravity of the two teeth numbered 6.

The mandibular pose difference calculated above has limited medical reference value, and a value with greater medical reference value can be calculated based on the result of the overall registration.

In an embodiment, a reference coordinate system may be calculated based on the dento-jaw (including the maxillary dentition and mandibular dentition), and the mandibular pose difference calculated above is decomposed in the reference coordinate system to obtain the translation and rotation components along each coordinate axis of the reference coordinate system.

In an embodiment, the normal direction of an occlusal surface and midline of the first three-dimensional digital model may be calculated, and the normal direction of the occlusal surface is used as the z-axis of the reference coordinate system, and the midline is used as the y-axis of the reference coordinate system. The x-axis of the reference coordinate system is perpendicular to the z-axis and the y-axis.

The translation component T of the pose difference is decomposed in the reference coordinate system to obtain the translation components in the front-back, left-right and up-down directions. The rotation component M is decomposed in the reference coordinate system to obtain the rotation components of pitch, roll and yaw. The components obtained after the pose difference is decomposed in the reference coordinate system are more intuitive in medicine and have more reference significance.

In another embodiment, a position difference between key points selected from the mandibular portions of the first and second three-dimensional digital models may be calculated based on the result of the overall registration of the maxillary portions of the first and second three-dimensional digital models. The position difference of these key points is more clinically instructive.

In an embodiment, the teeth of the maxilla and mandible may be divided into four quadrants, the teeth on the left half of the maxilla is in quadrant 1, the teeth on the right half of the maxilla is in quadrant 2, the teeth on the left half of the mandible is in quadrant 3, and the teeth on the right half of the mandible is in quadrant 4. For the teeth in each quadrant, they are numbered in order from the front teeth to the back teeth. For ease of explanation, the following description of the position difference between the key points is based on this numbering.

In an embodiment, the key points may include the center of gravity of the selected teeth, for example, the centers of gravity of teeth 31, 41, 33, 43, 36 and 46. Based on the position differences between the centers of gravity of these teeth in the first and second three-dimensional digital models, the difference in the local position of the dento-jaw may be intuitively understood.

In an embodiment, in order to at least partially eliminate errors caused by morphological differences, key points may be first selected in the first or second three-dimensional digital model, and then, based on the results of the above-mentioned fine registration of each tooth, these key points are projected onto the other one of the first and second three-dimensional digital models.

In another embodiment, the key point may include a midline point, which is the average of the midpoints of the incisal edges of tooth 31 and tooth 41. If the midline point moves downward, it indicates that the overbite is open. If the midline point moves leftward, it indicates that the anterior teeth of the mandibular are deviated to the left.

In another embodiment, the position differences of key points may be used in combination to represent the rotation of the mandible. For example, if tooth 36 moves downward and tooth 46 moves upward, it means that the mandible rotates leftward and rightward, that is, the mandible rotates around the y-axis.

It can be understood that the expression of jaw position difference based on the position difference of key points is not limited to the above examples.

The present application also provides a method for estimating tooth deviation, including followings.

Optionally, inputting data: two tooth models that have been registered. The two models may be located in the same coordinate system.

Optionally, the calculation of a point intersecting with the normal may include at least one of the following.

Optionally, determining the reference model and the comparison model: select one of the two models as the reference model (model A) and the other one of the two model as the comparison model (model B)

Optionally, setting threshold 1 and threshold 2.

Optionally, the point intersecting with the normal is determined by: selecting some or all points in model B, emitting bidirectional rays along the normal of each point (reference point), and finding an intersection point of the rays with model A.

Optionally, classification of intersection point distances: if a distance between a reference point and an intersection point is less than the threshold 1, it means that the two models are highly overlapped; if the distance between the reference point and the intersection point is between the threshold 1 and the threshold 2, it means that there is a certain difference between the two models; if the distance between the reference point and the intersection point is greater than the threshold 2 (or there is no intersection point), it means that the two models are very different and this part of model B has no corresponding relationship with model A.

Optionally, the determination of the distance threshold may include at least one of the following.

Optionally, threshold 1 is usually determined by the modeling accuracy, refer to the "point pair distance lower limit" mentioned above; for example, a single tooth model can take 0.1 mm.

Optionally, threshold 2 is usually determined by the upper limit of tooth morphological changes and should be greater than threshold 1; for example, if it is believed that the morphological changes (wear, etc.) of the teeth within a certain period of time will not exceed 0.5 mm, threshold 2 may be set to 0.5 mm.

Optionally, other thresholds between threshold 1 and threshold 2, it can be subdivided into multiple intervals to indicate the degree of tooth morphology change.

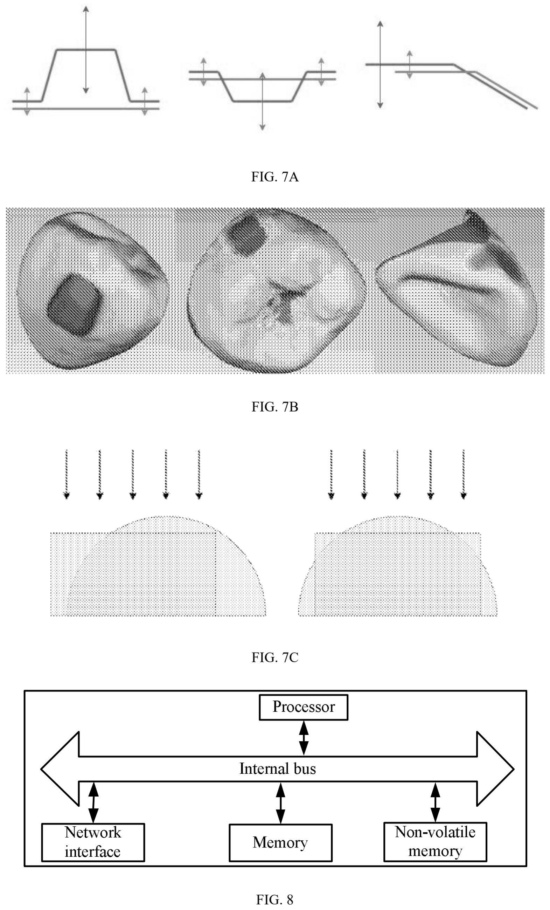

Optionally, several common results of the calculation of a point intersecting with the normal are shown in FIG. 7A. The green polyline segment represents the surface of the reference model, and the orange polyline segment represents the surface of the comparison model; the green arrow indicates that there is an intersection point, and the distance is less than threshold 1; the red arrow indicates that there is an intersection point, and the distance is between threshold 1 and threshold 2; the gray arrow indicates that there is no intersection point. On the left: the gray arrow exists in the internal area of the model. In the middle: the red arrow exists in the internal area of the model. On the right: the gray arrow exists in an edge area of the model.

The classification and processing of detection results may include at least one of the following: attachment information, tooth morphology variation information, and gum line variation information.

Optionally, the attachment information may include at least one of the following.

Optionally, in orthodontic treatment, some attachments with clinical functions are often attached to the surface of the crown; when re-establishes the tooth model for the patient, some attachments may still remain on the crown, and the model established at this time has obvious morphological differences compared with the tooth without the attachments; the removing the morphological differences caused by the attachments are the prerequisite for subsequent treatment estimation, plan improvement and other steps

Optionally, detection of attachments: in the classification of distance, if it belongs to the third category (there is a very large difference) and an area where the difference occurs is located inside the model, it is judged as an attachment; the attachment area is classified according to connectivity to obtain several unconnected areas on the tooth, corresponding to several different attachments.

Optionally, the tooth morphology variation information may include at least one of the following

Optionally, tooth morphology may change over time, usually as a result of wear.

Optionally, detection of tooth morphology changes: in the distance classification, if it belongs to the second category (there is a certain difference), and the area where the difference occurs is located inside the model, it is judged as a tooth morphology change.

Optionally, the gum line variation information may include at least one of the following.

Optionally, the clinical boundary between the crown and the gum is called the gum line, and its shape may change over time and may appear to be raised or lowered.

Optionally, during the tooth modeling process, the distance between the adjacent surfaces of adjacent teeth is close to zero, and the equipment cannot collect data, resulting in data missing; during orthodontic treatment, if a tooth is moved, a tooth is extracted, etc., the adjacent surfaces of the teeth that were originally missing data may be able to collect data in subsequent steps, and the result is also manifested as changes in the gum line.

Optionally, during the eruption of permanent teeth during the mixed dentition period, the crowns of the teeth continue to rise relative to the gums, resulting in changes in the gum line.

Optionally, detection of gum line changes: In distance classification, if it belongs to the third category (there is a very large difference) and the area where the difference occurs is at the edge of the model, it is judged as a gum line change.

FIG. 7B shows the results of the detection of attachments, tooth morphology changes, and gum line changes. Green indicates that the models are highly overlapped (less than threshold 1), light blue and yellow indicate that the models are slightly different (between threshold 1 and threshold 2 and closer to threshold 1), dark blue and red indicate that the models are highly different (between threshold 1 and threshold 2 and closer to threshold 2), and gray indicates that the models have no corresponding part (greater than threshold 2 or no intersection point). On the left: the gray area inside the tooth is judged as attachment; in the middle: the red and yellow area on the occlusal surface of the tooth is judged as wear; and on the right: the gray area at the edge of the tooth is judged as the change in the gum line.

Optionally, the attachment location correction information may include at least one of the following.

Optionally, when attaching attachments during orthodontic treatment, there will inevitably be a certain amount of error, resulting in a difference between an attachment in the digital model and an attachment the actual attachments.

Optionally, after completing the detection of the attachment, if there is information about the attachment in the patient's treatment plan, the positioning of the attachment in the treatment plan may be corrected based on the actual tooth model.

Optionally, inputting data: digital model of attachments (model C), tooth model without attachments (model A) and tooth model with attachments (model B).

Optionally, the model A and model B have been completely registered, and therefore are located in the same coordinate system; model C is a digital design performed on model A, and therefore is located in the same coordinate system as model A; therefore, model A, model B, and model C are all located in the same coordinate system.

Optionally, the bottom surface of model C is used as a reference surface, and after it is rasterized, rays are emitted along the normal direction to respectively find the nearest intersection points with model B and model C.

Optionally, a distance between two intersection points corresponding to each ray is calculated, and distances corresponding to all rays are summed up, and a result obtained is the objective function.

Optionally, the objective function can be a correlation between model C and model B, such as the proportion of overlapping volumes.

Optionally, the bottom surface of the model C is moved along a rasterization direction, and the above process is repeated. If the objective function becomes smaller, the movement in the rasterization direction is updated to the bottom surface of the model C.

Optionally, when the four possible directions in which the model C may move do not make the objective function smaller, the search is stopped and the model C is positioned to a position corresponding to the bottom surface.

FIG. 7C shows the correction of the attachment positioning. The green model represents the digital model of the attachment, the orange model represents the protrusion caused by the attachment on the actual tooth model, and the black arrow represents the direction of the ray after rasterization. On the left side: there is a certain offset between the green and orange models, and the distance calculated along the direction of the black arrow is larger. On the right side: The green model is relatively shifted to the right, and the distance calculated along the direction of the black arrow reaches the minimum value, which is the corrected positioning position of the digital model of the attachment.

In the method for estimating tooth deviations provided by the present application, the best registration between two tooth models may be found through adaptive parameter adjustment. Based on this registration, differences in attachments, tooth morphology changes, gum line changes, etc., are further found. The difference in tooth morphology is automatically detected with high efficiency and accuracy, eliminating interference from subjective factors. The differences in tooth morphology are automatically analyzed and divided into several common situations, providing a basis for subsequent processing. The errors in attachment positioning are automatically corrected, providing a basis for subsequent processing.

The embodiments of the present application provide a display interface of a method for estimating tooth deviation. The tooth deviation estimation may be implemented as a functional module in the software interface. For example, the functional module can be activated after the patient's orthodontic appliance is shipped. The doctor or user can operate the functional module to trigger the functional module.

FIG. 5A shows a display interface of a function module of tooth movement deviation analysis displayed in a software interactive interface after the patient's orthodontic appliance is delivered. This function module may be displayed in a sub-interface of medical record monitoring.

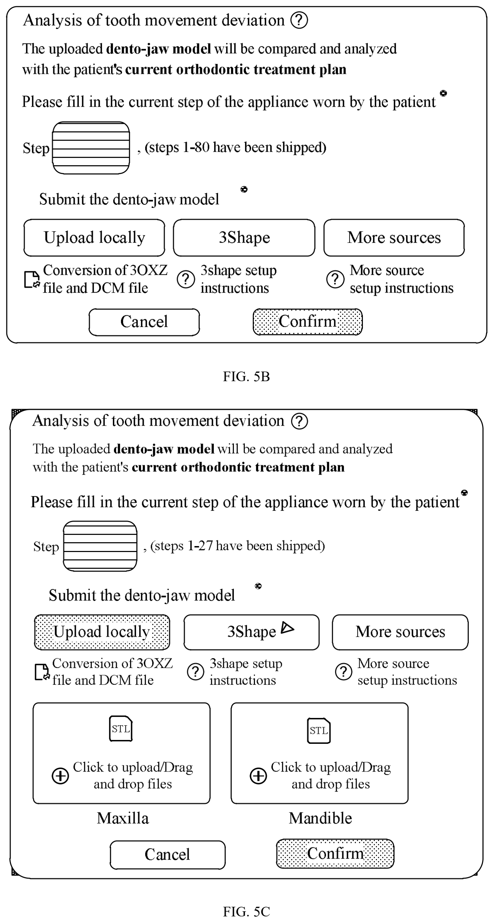

In response to a trigger instruction of the function module of the tooth movement deviation analysis, after triggering the function module, a data collection interface of the tooth movement deviation analysis may be jumped to. As shown in FIG. 5B and FIG. 5C, in response to an operation of a user, the step number currently worn by the patient may be obtained, and the patient's current dento-jaw model may be collected, which may be a three-dimensional digital model of the teeth obtained by a digital mouth scanning tool, or may be obtained through other collection devices, which is not limited here.

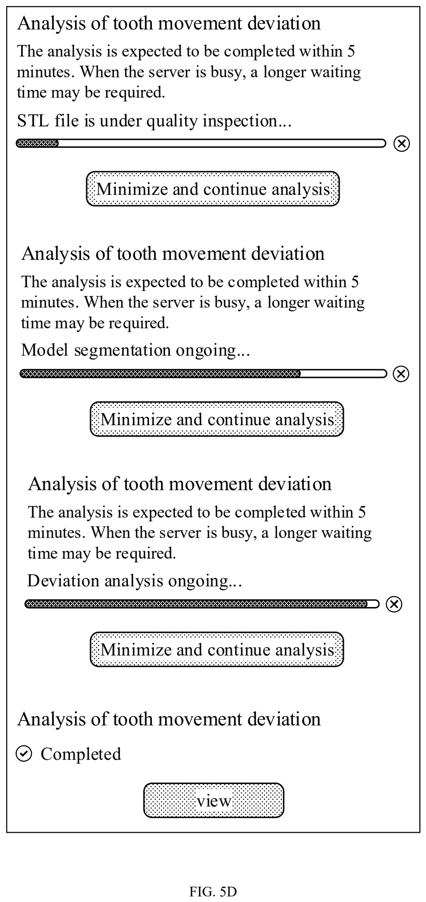

In response to a confirmed operation instruction of a collection interface, the tooth movement deviation analysis stage may be entered, as shown in FIG. 5D. The analysis stage includes the preprocessing of the uploaded file, for example, the quality inspection of the STL file. It may also include operations such as the segmentation of the three-dimensional digital model to obtain the registration result of the model. After obtaining the registration result, the tooth movement deviation analysis stage may be entered to obtain the estimation result of the tooth to be estimated.

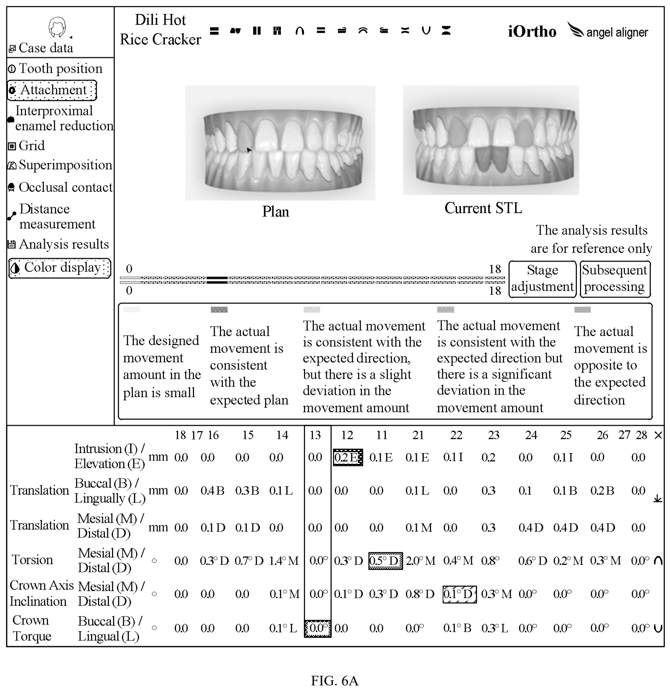

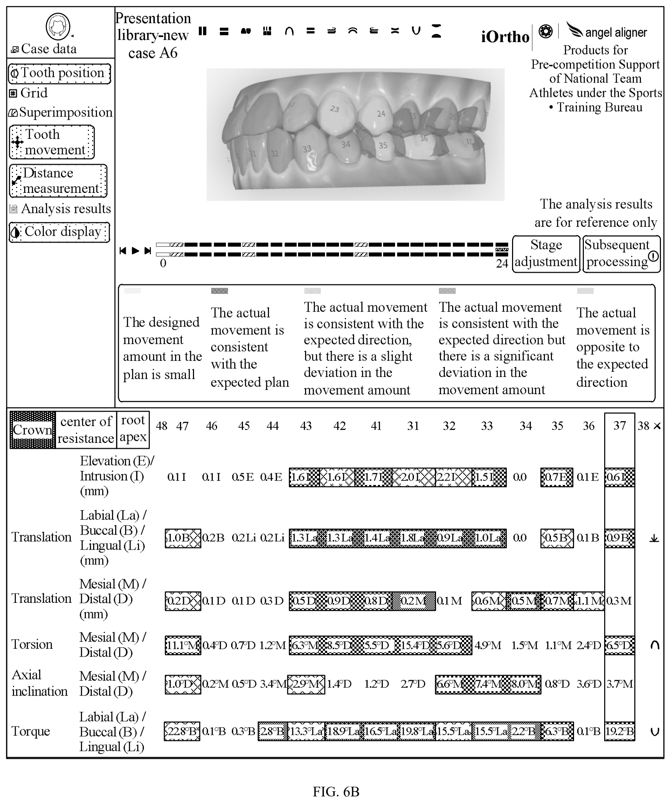

After the analysis is completed, the viewing page may be entered to view the estimation result. There are many ways to view, and the following examples are provided. FIG. 6A shows a deviation analysis result of a single tooth provided by the embodiments of the present application. For example, the first and second three-dimensional digital models and estimation information may be displayed on a first display interface. Optionally, the estimation information may include: the estimation result and a display method of the estimation result.

Optionally, the display interface may include a display of the first three-dimensional digital model and the second three-dimensional digital model to be estimated, and estimation information of a single tooth.

Optionally, the first and second three-dimensional digital models are displayed side by side in a display frame of the display interface.

Estimation information may be displayed in a various ways, including, for example, presenting the actual intraoral situation + data comparison of the design plan (transverse, sagittal, vertical, gap analysis).

Optionally, different colors can be displayed based on the estimation results of tooth movement to indicate different deviation degrees, and the estimation information further includes at least one of the following:

color markers included in the first and second three-dimensional digital models; here the color markers are used to identify a deviation degree of teeth;
estimation result including estimation information of at least one tooth to be estimated; herein the estimation information includes a display of color markers corresponding to the degree deviation of the teeth.

A color is used to mark teeth and add color annotations. This helps doctors to clearly understand which teeth they should focus on by the depth of color, thus improving the doctor's inspection efficiency.

For example, according to the degree of tooth movement deviation, the teeth are marked with colors (green, yellow, orange, and purple), representing the deviation degree from low to high and different deviation directions.

White (i.e. normal color of the crown) indicates that a designed movement amount in the plan is small; green indicates that the actual movement is consistent with the expected plan; yellow indicates that the actual movement is consistent with the expected direction, but there is a slight deviation in the movement amount; orange indicates that the actual movement is consistent with the expected direction, but there is a significant deviation in the movement amount; purple indicates that the actual movement is opposite to the expected direction.

Correspondingly, in the numerical table showing the estimation results corresponding to each tooth, a corresponding mark may represent based on corresponding color to display corresponding deviation information. By directly and horizontally comparing the initial position, current dentition, expected effect of the plan, the horizontal, sagittal and vertical of the target position, and the gap analysis, it is convenient for doctors to intuitively understand the effect achieved by the current patient after wearing the orthodontic appliance and understand the progress of the case.

Optionally, in response to a user clicking operation on a step number, the display may be switched to display a first three-dimensional digital model corresponding to the step number and corresponding estimation information.

Optionally, in order to allow the doctor to focus on the patient's current dentition, the plan is superimposed on the current dentition in a gray semi-transparent state. By dragging the plan step bar, the doctor can easily understand at which step the patient began to deviate from the orthodontic appliance.

In some embodiments, in response to an superimpose display operation of a third three-dimensional digital model, a three-dimensional digital model is displayed on the display interface, and the three-dimensional digital model is used to display: the first or second three-dimensional digital model, and the third three-dimensional digital model; the digital model displays color markers indicating a deviation degree of tooth, and teeth of the third three-dimensional digital model and teeth of the first or second three-dimensional digital model have different display modes.

In a possible implementation, the design plan (first three-dimensional digital model) in a gray semi-transparent state may be superimposed on the current dentition (second three-dimensional digital model). By dragging the plan step bar, the doctor can easily understand the step at which the patient began to deviate from the orthodontic appliance.

For example, in response to the superimpose operation from a user, for example, the superimpose plan may be clicked by the user to display the tooth model, and the tooth model displays: the design plan is superimposed on the patient's current dentition model obtained by intraoral scanning, and the color representation corresponding to the deviation is displayed at the same time, and the design plan is translucent gray.

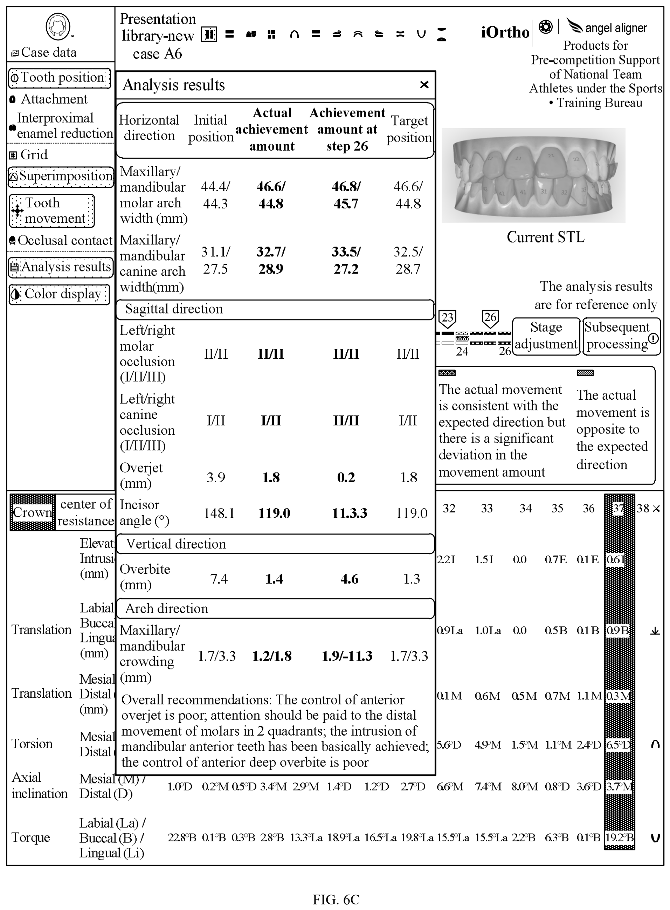

Optionally, as shown in FIG. 6C, jaw position deviation information, step deviation information, etc. may be displayed in the form of a floating window.

Optionally, the floating window and the three-dimensional digital model are displayed simultaneously, and in the floating window state, the number of clicks of the doctor or user may be reduced, or the number of interactions of the doctor or user may be reduced, so that the doctor or user may make a comprehensive judgment on tooth deviation based on multiple types of information, thereby improving viewing efficiency and viewing effect.

Optionally, the three-dimensional tooth model may also include tooth root information. In this case, the patient's tooth root information may also be displayed in the displayed digital model, thereby displaying comparative information of teeth roots.

Root comparison: by supplementing the uploaded STL with the CBCT file, and the root with the dento-jaw can be combined. By comparing the designed root or virtual root with the actual root, the root torsion and the current tooth status can be understood. The specific comparison method can refer to the estimation method in the above embodiment, which will not be repeated here.

FIG. 8 shows a schematic structural diagram of a system for estimating a tooth deviation in the embodiments of the present application, which may include a storage device and a processor. The storage device stores a computer program for estimating the tooth deviation. When the computer program is run by the processor, possible method described in the above embodiments may be executed.

The system may also include a computing device, for example, the computing device may have multiple components coupled thereto. The computing device may include a processor and a memory. The memory may have various types of information, including data and executable instructions.

The system may also include: an example computing device readable medium including executable instructions, the executable instructions being executable by the processor to perform a method according to one or more embodiments of the present application.

The processor may execute instructions stored on an internal or external non-transitory computing device readable medium (CRM). For example, the non-transitory CRM may include volatile and/or non-volatile memory. Volatile memory may include memory that relies on power to store information, such as various types of dynamic random access memory (DRAM), etc. Non-volatile memory may include memory that does not rely on power to store information.

In some embodiments, the memory and/or processor may be located on the computing device or separately from the computing device. Thus, in the embodiments as shown in FIG. 8, the computing device may include a network interface. Such an interface may allow processing to be performed on another networked computing device, may be used to obtain information about the patient, and/or may be used to obtain data and/or executable instructions for various embodiments provided herein.

As shown in the embodiment of FIG. 7, for example, a computing device may include one or more input and/or output interfaces. Such interfaces may be used to connect the computing device to one or more input and/or output devices.

For example, in the illustrated embodiment, the input and/or output devices may include a scanning device, a camera interface, an input device (e.g., a mouse, a keyboard, etc.), a display device (e.g., a display, etc.), a printer, and/or one or more other input devices. The input/output interface may receive executable instructions and/or data, which may be stored in a data storage device (e.g., a memory), representing a tooth model of a patient's dentition.

In some embodiments, the scanning device may be configured to scan one or more physical dental models of the patient's dentition. In one or more embodiments, the scanning device may be configured to directly scan the patient's dentition and/or dental appliances. The scanning device may be configured to input data into a computing device.

In some embodiments, the camera interface may receive input from an imaging device (e.g., a 2D or 3D imaging device), such as a digital camera, a printed photo scanner, and/or other suitable imaging device. For example, the input from the imaging device may be stored in a memory.

The processor may execute instructions to present and provide a tooth deviation estimation result on a display. The computing device may be configured to allow a treating professional or other user to enter tooth information of a tooth to be estimated. The received input may be sent to the processor as data and/or may be stored in a memory.

Such connectivity may allow for the input and/or output of data and/or instructions and other types of information. Some embodiments may be distributed among various computing devices within one or more networks.

A processor associated with a data storage device (e.g., a memory) may be associated with data. The processor associated with the memory may store and/or utilize data and/or execute instructions for obtaining first and second three-dimensional digital models, and obtaining estimation information of at least one tooth to be estimated based on a registration result of at least one tooth to be estimated in the first and second three-dimensional digital models. Such data may include the first and second three-dimensional digital models.

The processor may cause the computing device to execute a method, which includes, for example, executing a method for estimating a tooth deviation. In various embodiments of the present disclosure, the processor coupled to the memory may cause the computing device to execute the method, which includes obtaining first and second three-dimensional digital models, and obtaining estimation information of at least one tooth to be estimated based on a registration result of at least one tooth to be estimated of the first and second three-dimensional digital models.

This embodiment provides a computer-readable storage medium on which a computer program is stored. When the computer program is executed by a processor, the method in the above embodiment is implemented.

Although specific embodiments have been illustrated and described herein, those of ordinary skill in the art will appreciate that any arrangement calculated to achieve the same technique may be substituted for the specific embodiments shown. This disclosure is intended to cover any and all modifications or variations of the various embodiments of the disclosure.

It should be understood that the above description is made in an illustrative manner, not a restrictive one. By reading the above description, those skilled in the art will understand the combination of the above embodiments and other embodiments not specifically described herein. The scope of the various embodiments of the present disclosure includes any other application using the above structures and methods. Therefore, the scope of the various embodiments of the present disclosure should be determined with reference to the attached claims and all equivalent scopes to which these claims are entitled.

In the foregoing detailed description, various features are grouped together in the example embodiments shown in the figures for the purpose of simplifying the disclosure. The method of the present disclosure should not be interpreted as reflecting an intention that the embodiments of the present disclosure require more features than those expressly recited in each claim. On the contrary, as reflected in the following claims, the inventive subject matter does not lie in all features of a single embodiment of the present disclosure. Therefore, the following claims are hereby incorporated into the detailed description, with each claim standing on its own as a separate embodiment.

Unless expressly stated otherwise, terms and phrases used herein, and variations thereof, should be construed as open ended as opposed to limiting. In some embodiments, the appearance of expansive words and phrases such as "one or more", "at least", "but not limited to", or other similar terms should not be understood as intending or requiring a narrowing in examples where such expansive words may not be present.

**Claims**

**1.** A method for estimating tooth deviation, comprising:

obtaining first and second three-dimensional digital models; wherein the first and second three-dimensional digital models represent different three-dimensional digital models of a same patient, the second three-dimensional digital model is a three-dimensional digital model obtained by scanning teeth of the patient, and the first three-dimensional digital model is one of a series of successive three-dimensional digital models in an orthodontic treatment plan using a shell-style orthodontic appliance;

obtaining a first reference point set and a second reference point set based on the first and second three-dimensional digital models; wherein the first reference point set comprises a reference point of at least one tooth to be estimated of the first three-dimensional digital model, and the second reference point set comprises a reference point of the at least one tooth of the second three-dimensional digital model corresponding to the reference point in the first reference point set;

preforming registration between the at least one tooth to be estimated of the first three-dimensional digital model and the at least one tooth to be estimated of the second three-dimensional digital model, based on the first reference point set and second reference point set; wherein a result of the registration is used to estimate a deviation of the at least one tooth to be estimated.

2. The method according to claim 1, wherein a reference point pair of the at least one tooth to be estimated is registered after being weighted; a weight of the reference point pair is determined based on at least one of following factors: a designed movement amount of a tooth, a registration confidence level of the tooth corresponding to a reference point, a degree of difficulty in tooth movement, a position of the reference point on the tooth, or a preset weight.

3. The method according to claim 1 or 2, wherein a weight of at least one reference point pair of the at least one tooth to be estimated is assigned according to at least one of following:

(1) assigning the weight according to a designed movement amount of each tooth in a treatment step corresponding to the second three-dimensional digital model; wherein the smaller the designed movement amount of the tooth corresponding to a reference point, the greater the weight;

(2) assigning the weight according to a registration confidence level of the tooth corresponding to the reference point; wherein the higher a fine registration confidence level of the tooth corresponding to the reference point, the greater the weight;

(3) assigning the weight according to a degree of difficulty in tooth movement; wherein the easier the movement of the tooth corresponding to the reference point is achieved, the greater the weight; or

(4) assigning the weight according to a position of the reference point on the tooth, wherein the closer the position to a crown, the greater the weight.

4. The method according to any one of claims 1-3, wherein a plurality of second three-dimensional digital models are provided, and the second three-dimensional digital model corresponding to estimation information of the at least one tooth to be estimated is determined according to at least one of following:

step information specified by a user;

estimated step information obtained from a result of registration between the first three-dimensional digital model and second three-dimensional digital models corresponding to multiple steps; or

differences between each of the second three-dimensional digital models corresponding to multiple steps and the first three-dimensional digital model.

5. The method according to any one of claims 1-4, further comprising:

performing optimization processing based on a plurality of registration results each for a single tooth, to determine a registration result of multiple teeth to be estimated; wherein the single tooth is a tooth in the multiple teeth to be estimated, one of the plurality of registration results is a registration result between reference points of first and second three-dimensional digital models corresponding to one single tooth.

6. The method according to any one of claims 1-5, further comprising:

determining distance information between a reference point of the second three-dimensional digital model and the first three-dimensional digital model based on normal distribution model data of the first three-dimensional digital model;

determining distance information between the first and second three-dimensional digital models according to distance information of each reference point; and

determining a registration result of the tooth to be estimated according to the distance information between the first and second three-dimensional digital models.

7. The method according to any one of claims 1-6, further comprising:

performing a weighting process according to a registration result for each tooth to obtain registration reference information of teeth to be registered; and

determining a registration result of the teeth to be estimated by comparing the registration reference information of the teeth to be registered with a plurality of registration results each for a single tooth.

8. The method according to any one of claims 1-7, further comprising:
determining a registration result of teeth to be estimated of the first and second three-dimensional digital models based on a registration confidence level or at least one registration confidence level of at least one tooth in the teeth to be estimated.

9. The method according to claim 8, wherein the registration confidence level is determined based on at least one of following:

a proportion of registered reference point pairs in all reference point pairs;

residual information of registration for a single tooth;

residual information of registration for at least one tooth to be estimated;

estimated step information and step information corresponding to collection of the first three-dimensional digital model;

initial step number and final step number;

distance information of registration for the single tooth; or

distance information of registration for the at least one tooth to be estimated.

10. The method according to any one of claims 1-9, further comprising:

obtaining a registration result of a single tooth in teeth to be estimated; wherein the registration of the single tooth is determined based on registration of a reference point pair corresponding to the single tooth; and

performing registration between the teeth to be estimated of the first and second three-dimensional digital models according to the registration result of the single tooth in the teeth to be estimated and the reference point pair formed by the first reference point set and the second reference point set.

11. The method according to any one of claims 1-10, wherein a reference point pair corresponding to a single tooth is a weighted reference point pair during a registration process, and a weight of the reference point pair is determined based on at least one of following factors:
tooth position information, tooth position feature information, relative information of a distance between reference points of the reference point pair, or a threshold condition satisfied by the distance of the reference points of the reference point pair.

12. The method according to any one of claims 1-11, wherein a weight of a reference point pair of a single tooth is assigned according to at least one of following:

(1) assigning the weight according to a long-axis coordinate of a local coordinate system, wherein: a reference point pair closer to an incisal edge or occlusal surface of the tooth has a higher weight, and a reference point pair closer to a gum line has a lower weight;

(2) assigning the weight according to reference point pairs sorted by distance, wherein: in each iteration, the reference point pairs are sorted from large to small by distance, and a reference point pair with a larger distance has a lower weight; or

(3) assigning a weight according to a distance threshold, wherein: in each iteration, based on that a distance of a reference point pair exceeds a preset distance threshold, a weight of the reference point pair is reduced.

13. The method according to any one of claims 1-12, wherein a reference point is determined according to at least one of the following:

coordinate points of the local coordinate system of the tooth;

feature points of the tooth; or

sampling points of a tooth model; wherein the tooth model is one of the first and second three-dimensional digital models.

14. The method according to any one of claims 1-13, wherein a reference point pair is determined according to at least one of following:

for a reference point of one tooth model, determining a reference point of another tooth model according to coordinate information of the one tooth model or tooth feature point information of the one tooth model to form the reference point pair;

for a reference point of one tooth model, finding an intersection point with another one tooth model by casting a ray from the reference point along a normal direction, wherein a ray origin and the intersection point form a corresponding reference point pair; or

for a reference point of one tooth model, finding a point of another tooth model closest to the reference point, wherein the reference point and the point closest to the reference point form a corresponding point pair;

wherein the tooth model is one of the first and second three-dimensional digital models.

15. The method according to any one of claims 1-14, wherein the registration is performed based on iteration, and the iteration is stopped when reference points of a reference point pair for registration meet an iteration condition.

16. The method according to any one of claims 1-15, wherein the iteration condition comprises at least one of following:

a calculated distance of a point pair meets a distance threshold condition;

a proportion of fully registered point pairs meets a proportion threshold condition;

a number of iteration steps meets a maximum number of iterations;

a minimum update difference meets an update threshold condition; or

a registration confidence level meets a confidence level threshold condition.

17. The method according to any one of claims 1-16, wherein a registration result of a single tooth comprises a registration result between at least one pair of first and second three-dimensional digital models, and a registration result of each pair of tooth models comprises at least one of following:

each pair of tooth models is located in a same coordinate system after registration;

a confidence level of registration result of each pair of teeth; or

an anomalous point in the registration result of each pair of teeth.

18. The method according to any one of claims 1-17, wherein the registration result further comprises: estimated step information of the tooth to be estimated, and the estimated step information is determined based on a registration result obtained by performing registration between the second three-dimensional digital model and at least one first three-dimensional digital model.

19. The method according to any one of claims 1-18, wherein the first three-dimensional digital model is one of the series of successive three-dimensional digital models that is closest to the second three-dimensional digital model.

20. The method according to any one of claims 1-19, further comprising:

obtaining estimation information of the at least one tooth to be estimated based on a registration result of the at least one tooth to be estimated between the first and second three-dimensional digital models;

wherein the estimation information of the at least one tooth to be estimated comprises at least one of the following: an absolute change amount of a single tooth, a relative change amount of the single tooth, jaw position deviation information of the at least one tooth to be estimated, and step number deviation information of the at least one tooth to be estimated.

21. The method according to any one of claims 1-20, wherein

a relative change amount of a single tooth is determined based on a designed movement amount and an actual movement amount of the tooth;

the designed movement amount of the tooth is determined based on a registration result of a tooth model obtained by scanning the tooth at a corresponding time; and

the actual movement amount is determined based on a registration result of the first and second three-dimensional digital models corresponding to the tooth.

22. The method according to any one of claims 1-21, wherein the estimation information comprises an estimation result of a single tooth, and the estimation result of the single tooth comprises at least one of following:

a total tooth movement distance d, a translation amount dx in a mesiodistal direction, a translation amount dy in a labiolingual direction, a translation amount dz in a vertical direction, a total tooth rotation angle R, torque Rx, axial tilt Ry, and torsion Rz.

23. The method according to any one of claims 1-22, wherein the estimation information comprises jaw position deviation information, the jaw position deviation information comprises a translation amount and a rotation amount, and the jaw position deviation information of the at least one tooth to be estimated is determined in following manner:

obtaining first reference jaw position deviation information according to a first three-dimensional digital model of a first jaw position of the patient and a first three-dimensional digital model of a second jaw position of the patient;
obtaining second reference jaw position deviation information according to a second three-dimensional digital model of the first jaw position of the patient and a second three-dimensional digital model of the second jaw position of the patient;
obtaining a registration result of the at least one tooth according to the first three-dimensional digital model of first jaw position of the patient and the second three-dimensional digital model of the first jaw position of the patient and determining translation information and rotation information of the at least one tooth;
obtaining the jaw position deviation information of the at least one tooth to be estimated according to the translation information and rotation information of the at least one tooth and the first and second reference jaw position deviation information.

24. The method according to any one of claims 1-23, wherein

estimation information comprises jaw position deviation information, the jaw position deviation information comprises deviation information of a key tooth position, and
the deviation information of the key tooth position comprises: translation change amount between an actual state of a key tooth and a designed state of the key tooth.

25. The method according to any one of claims 1-24, wherein estimation information comprises jaw position deviation information, and the jaw position deviation information comprises deviation information corresponding to a clinical indicator.

26. The method according to any one of claims 1-25, wherein the clinical indicator comprises at least one of following: anterior overjet, anterior overbite, posterior overjet, posterior overbite, sagittal relationship of molars, and a molar occlusion depth.

27. The method according to any one of claims 1-26, wherein the estimation information comprises jaw position deviation information, and the method further comprises:

obtaining a reference point of a clinical indicator;
determining jaw position information of the clinical indicator of the first three-dimensional digital model according to a reference point of the clinical indicator corresponding to the first three-dimensional digital model;
determining jaw position reference information of the clinical indicator of the second three-dimensional digital model according to a reference point of the clinical indicator corresponding to the second three-dimensional digital model; and
determining jaw position deviation information according to the jaw position information of the clinical indicator and the jaw position reference information of the clinical indicator.

28. The method according to any one of claims 1-27, further comprising:

obtaining a change amount of a tooth in a plurality of digital models corresponding to a plurality of steps;
determining step deviation information of the tooth according to a distance between estimated step information and actual step information.

29. The method according to any one of claims 1-28, further comprising:
displaying the first and second three-dimensional digital models and the estimation information on a first display interface.

30. The method according to any one of claims 1-29, wherein the estimation information further comprises at least one of following:

color markers comprised in the first and second three-dimensional digital models, wherein the color markers are used to identify a deviation degree of teeth; or
estimation information of the at least one tooth to be estimated, wherein the estimation information comprises a display of color markers corresponding to the deviation degree of the teeth.

31. The method according to any one of claims 1-30, further comprising:
displaying the first and second three-dimensional digital models side by side in one display frame of a display interface.

32. The method according to any one of claims 1-31, further comprising:
in response to a superimpose display operation of a third three-dimensional digital model, displaying a three-dimensional digital model on a display interface; wherein the three-dimensional digital model is used to display the second and third three-dimensional digital models, color markers indicating a deviation degree of teeth are displayed on the digital models, a display mode of teeth of the third three-dimensional digital model is different from a display mode of teeth of the first or second three-dimensional digital model, the third three-dimensional digital model is one of the series of successive three-dimensional digital models in the orthodontic treatment plan using the shell-style orthodontic appliance, and the third three-dimensional digital model corresponds to a different step from the first three-dimensional digital model.

33. The method according to any one of claims 1-32, wherein the estimation result comprises at least one of following: attachment information of teeth, tooth morphology variation information, and gum line variation information.

34. A computer-implemented method for calculating an achievement rate of orthodontic treatment, comprising:

obtaining a registration result of a tooth to be estimated between a three-dimensional digital model representing an initial tooth arrangement of a dentition and a three-dimensional digital model representing a current state of the dentition by using the method according to any one of claims 1-33, wherein the registration result comprises a first pose difference of the tooth to be estimated, and the first pose difference is decomposed in a reference coordinate system;
measuring and obtaining a registration result between the three-dimensional digital model representing the initial tooth arrangement of the dentition and a three-dimensional digital model representing a target tooth arrangement of the dentition; wherein the registration result comprises a second pose difference of the tooth to be estimated, and the second pose difference is decomposed in the reference coordinate system; and
dividing a component of the first pose difference by a corresponding component of the second pose difference to obtain an achievement rate of the tooth to be estimated in a corresponding movement direction.

35. A computer system for estimating tooth deviation, comprising a storage device and a processor, wherein the storage device stores a computer program for estimating tooth deviation, and when the computer program is run by the processor, the processor executes the method according to any one of claims 1-34.

Obtaining first and second three-dimensional digital models — 110

↓

Obtaining a first reference point set and a second reference point set according to the first and the second three-dimensional digital models — 120

↓

Based on the first reference point set and the second reference point set, performing registration between at least one tooth to be estimated of the first three-dimensional digital model and at least one tooth to be estimated of the second three-dimensional digital model — 130

FIG. 1

FIG. 2A

FIG. 2B

FIG. 3A

FIG. 3B

FIG. 4A

FIG. 4B

FIG. 4C

| iOrtho Workbench Case Patient Example | Angel Academy | 🔍 Search patient name/notes | ✎ Excellent cases | ✳ 📢 Chinese 👤 |
|---|---|---|---|---|

Institution name: Yang Guoguo Institution 2

Presentation library-new case A6 ☆

✓ 🅰 Male   23 years old
Undergoing orthodontic treatment
⛺ Protrusion

▶ demonstration

✎ Add remarks

The new features of iOrtho ✳ in October have been launched, check now ✕

Case data

| Database 2 Group | Orthodontic instructions 1 | Case data 0 |
|---|---|---|

Design phase [Confirmed] 3D ↩↪

19-09-2023 Scheme 1

Case monitoring ⑦ User Guide | View monitoring data

Edit
Expected date to finish wearing the appliance   10-07-2024
Appliance issuance date   19-12-2023
Aligner issuance   Steps 11-27
Wearing requirements   7=5days per step

| Add monitoring operation | Add deviation analysis |
|---|---|

Appliance processing   ⑦ Help guide

| 🔄 Stage adjustment | ✓ Complete case | 🛠 ⓘ Subsequent processing |
|---|---|---|

Case progress

Production phase - First shipment   ⌃

Logistics information   View more

Shipped

Maxilla (1-27)
Mandible (1-27)

Design phase--Appliance

Shipment delivered to DHL
19-09-2023 11:42:44

FIG. 5A

Analysis of tooth movement deviation ⑦

The uploaded **dento-jaw model** will be compared and analyzed with the patient's **current orthodontic treatment plan**

Please fill in the current step of the appliance worn by the patient °

Step [____] , (steps 1-80 have been shipped)

Submit the dento-jaw model °

| Upload locally | 3Shape | More sources |

⤷ Conversion of 3OXZ file and DCM file    ⑦ 3shape setup instructions    ⑦ More source setup instructions

| Cancel | Confirm |

FIG. 5B

Analysis of tooth movement deviation ⑦

The uploaded **dento-jaw model** will be compared and analyzed with the patient's **current orthodontic treatment plan**

Please fill in the current step of the appliance worn by the patient °

Step [____] , (steps 1-27 have been shipped)

Submit the dento-jaw model °

| Upload locally | 3Shape ▷ | More sources |

⤷ Conversion of 3OXZ file and DCM file    ⑦ 3shape setup instructions    ⑦ More source setup instructions

STL
⊕ Click to upload/Drag and drop files

STL
⊕ Click to upload/Drag and drop files

Maxilla          Mandible

| Cancel | Confirm |

FIG. 5C

Analysis of tooth movement deviation

The analysis is expected to be completed within 5 minutes. When the server is busy, a longer waiting time may be required.

STL file is under quality inspection...

⊗

Minimize and continue analysis

Analysis of tooth movement deviation

The analysis is expected to be completed within 5 minutes. When the server is busy, a longer waiting time may be required.

Model segmentation ongoing...

⊗

Minimize and continue analysis

Analysis of tooth movement deviation

The analysis is expected to be completed within 5 minutes. When the server is busy, a longer waiting time may be required.

Deviation analysis ongoing...

⊗

Minimize and continue analysis

Analysis of tooth movement deviation

⊘ Completed

view

FIG. 5D

**Case data**
- ⓘ Tooth position
- Attachment
- Interproximal enamel reduction
- Grid
- Superimposition
- Occlusal contact
- Distance measurement
- Analysis results
- Color display

Dili Hot Rice Cracker  ≡ ⏄ ‖ ⫙ ∩ ≡ ⇌ ⌢ ⇋ ≍ ∪ Σ    **iOrtho** ✎ angel aligner

Plan    Current STL

The analysis results are for reference only

0 ——————————————————— 18 | Stage adjustment | Subsequent processing
0 ——————————————————— 18

| The designed movement amount in the plan is small | The actual movement is consistent with the expected plan | The actual movement is consistent with the expected direction, but there is a slight deviation in the movement amount | The actual movement is consistent with the expected direction but there is a significant deviation in the movement amount | The actual movement is opposite to the expected direction |

|  |  |  | mm | 18 | 17 | 16 | 15 | 14 | 13 | 12 | 11 | 21 | 22 | 23 | 24 | 25 | 26 | 27 | 28 |
|---|---|---|---|---|---|---|---|---|---|---|---|---|---|---|---|---|---|---|---|
|  | Intrusion (I) / Elevation (E) | mm | | 0.0 | | 0.0 | 0.0 | 0.0 | 0.0 | 0.2 E | 0.1 E | 0.1 E | 0.1 I | 0.2 | 0.0 | 0.1 I | 0.0 | | 0.0 |
| Translation | Buccal (B) / Lingually (L) | mm | | 0.0 | | 0.4 B | 0.3 B | 0.1 L | 0.0 | 0.0 | 0.0 | 0.1 L | 0.0 | 0.3 | 0.1 | 0.1 B | 0.2 B | | 0.0 |
| Translation | Mesial (M) / Distal (D) | mm | | 0.0 | | 0.1 D | 0.1 D | 0.0 | 0.0 | 0.0 | 0.0 | 0.1 M | 0.0 | 0.3 | 0.4 D | 0.4 D | 0.4 D | | 0.0 |
| Torsion | Mesial (M) / Distal (D) | ° | | 0.0 | | 0.3° D | 0.7° D | 1.4° M | 0.0° | 0.3° D | 0.5° D | 2.0° M | 0.4° M | 0.8° | 0.6° D | 0.2° M | 0.3° M | | 0.0° |
| Crown Axis Inclination | Mesial (M) / Distal (D) | ° | | 0.0 | | 0.0 | 0.0 | 0.1° M | 0.0° | 0.1° D | 0.3° D | 0.8° D | 0.1° D | 0.3° M | 0.0° | 0.0° | 0.0° | | 0.0° |
| Crown Torque | Buccal (B) / Lingual (L) | ° | | 0.0 | | 0.0 | 0.0 | 0.1° L | 0.0° | 0.0 | 0.0 | 0.0° | 0.1° B | 0.3° L | 0.0° | 0.0° | 0.0° | | 0.0° |

FIG. 6A

Case data
Tooth position
Grid
Superimposition
Tooth movement
Distance measurement
Analysis results
Color display

Presentation library-new case A6

iOrtho — angel aligner

Products for Pre-competition Support of National Team Athletes under the Sports • Training Bureau

The analysis results are for reference only

0 | 24 | Stage adjustment | Subsequent processing

| The designed movement amount in the plan is small | The actual movement is consistent with the expected plan | The actual movement is consistent with the expected direction, but there is a slight deviation in the movement amount | The actual movement is consistent with the expected direction but there is a significant deviation in the movement amount | The actual movement is opposite to the expected direction |

| Crown center of resistance root apex | | | 48 | 47 | 46 | 45 | 44 | 43 | 42 | 41 | 31 | 32 | 33 | 34 | 35 | 36 | 37 | 38 × |
|---|---|---|---|---|---|---|---|---|---|---|---|---|---|---|---|---|---|---|
| | Elevation (E)/ Intrusion (I) (mm) | | 0.1 I | 0.1 I | 0.5 E | 0.4 E | 1.6 I | 1.6 I | 1.7 I | 2.0 I | 2.2 I | 1.5 I | 0.0 | 0.7 E | 0.1 E | 0.6 I | |
| Translation | Labial (La) / Buccal (B) / Lingual (Li) (mm) | | 1.0 B | 0.2 B | 0.2 Li | 0.2 Li | 1.3 La | 1.3 La | 1.4 La | 1.8 La | 0.9 La | 1.0 La | 0.0 | 0.5 B | 0.1 B | 0.9 B | ↓ |
| Translation | Mesial (M) / Distal (D) (mm) | | 0.2 D | 0.1 D | 0.1 D | 0.3 D | 0.5 D | 0.9 D | 0.8 D | 0.2 M | 0.1 M | 0.6 M | 0.5 M | 0.7 M | 1.1 M | 0.3 M | |
| Torsion | Mesial (M) / Distal (D) | | 11.1°M | 0.4°D | 0.7°D | 1.2°M | 6.3°M | 8.5°D | 5.5°D | 15.4°D | 5.6°D | 4.9°M | 1.5°M | 1.1°M | 2.4°D | 6.5°D | ∩ |
| Axial inclination | Mesial (M) / Distal (D) | | 1.0°D | 0.2°M | 0.5°D | 3.4°M | 2.9°M | 1.4°D | 1.2°D | 2.7°D | 6.6°M | 7.4°M | 8.0°M | 0.8°D | 3.6°D | 3.7°M | |
| Torque | Labial (La) / Buccal (B) / Lingual (Li) | | 22.8°B | 0.1°B | 0.3°B | 2.8°B | 13.3°La | 18.9°La | 16.5°La | 19.8°La | 15.5°La | 15.5°La | 2.2°B | 6.3°B | 0.1°B | 19.2°B | ∪ |

FIG. 6B

Case data

Presentation library-new case A6

iOrtho

angel aligner

Products for Pre-competition Support of National Team Athletes under the Sports • Training Bureau

Tooth position

Attachment

Interproximal enamel reduction

Grid

Superimposition

Tooth movement

Occlusal contact

Analysis results

Color display

**Analysis results** ✕

| Horizontal direction | Initial position | Actual achievement amount | Achievement amount at step 26 | Target position |
|---|---|---|---|---|
| Maxillary/ mandibular molar arch width (mm) | 44.4/ 44.3 | **46.6/ 44.8** | **46.8/ 45.7** | 46.6/ 44.8 |
| Maxillary/ mandibular canine arch width(mm) | 31.1/ 27.5 | **32.7/ 28.9** | **33.5/ 27.2** | 32.5/ 28.7 |

Sagittal direction

| | | | | |
|---|---|---|---|---|
| Left/right molar occlusion (I/II/III) | II/II | **II/II** | **II/II** | II/II |
| Left/right canine occlusion (I/II/III) | I/II | **I/II** | **II/II** | I/II |
| Overjet (mm) | 3.9 | **1.8** | **0.2** | 1.8 |
| Incisor angle (°) | 148.1 | **119.0** | **11.3.3** | 119.0 |

Vertical direction

| | | | | |
|---|---|---|---|---|
| Overbite (mm) | 7.4 | **1.4** | **4.6** | 1.3 |

Arch direction

| | | | | |
|---|---|---|---|---|
| Maxillary/ mandibular crowding (mm) | 1.7/3.3 | **1.2/1.8** | **1.9/-11.3** | 1.7/3.3 |

Overall recommendations: The control of anterior overjet is poor; attention should be paid to the distal movement of molars in 2 quadrants; the intrusion of mandibular anterior teeth has been basically achieved; the control of anterior deep overbite is poor

Current STL

The analysis results are for reference only

23 | 26

24 | 26

Stage adjustment | Subsequent processing

The actual movement is consistent with the expected direction but there is a significant deviation in the movement amount

The actual movement is opposite to the expected direction

Crown — center of resistance

Elevat Intrusi (mm)

Translation — Labial Bucca Lingua (mm)

Translation — Mesial Distal (mm)

Torsion — Mesial Distal

Axial inclination — Mesial (M)/ Distal (D)

Torque — Labial (La) / Buccal (B) / Lingual (Li)

| | 32 | 33 | 34 | 35 | 36 | 37 | 38 ✕ |
|---|---|---|---|---|---|---|---|
| | 2.2 I | 1.5 I | 0.0 | 0.7 E | 0.1 E | 0.6 I | |
| | 0.9 La | 1.0 La | 0.0 | 0.5 B | 0.1 B | 0.9 B | |
| | 0.1 M | 0.6 M | 0.5 M | 0.7 M | 1.1 M | 0.3 M | |
| | 5.6° D | 4.9° M | 1.5° M | 1.1° M | 2.4° D | 6.5° D | |
| 1.0° D 0.2° M 0.5° D 3.4° M 2.9° M 1.4° D 1.2° D 2.7° D | 6.6° M | 7.4° M | 8.0° M | 0.8° D | 3.6° D | 3.7° M | |
| 22.8° B 0.1° B 0.3° B 2.8° B 13.3° La 18.9° La 16.5° La 19.8° La 15.5° La | 15.5° La | 2.2° B | 6.3° B | 0.1° B | 19.2° B | |

FIG. 6C

FIG. 7A

FIG. 7B

FIG. 7C

FIG. 8

## INTERNATIONAL SEARCH REPORT

| | International application No. |
|---|---|
| | **PCT/CN2024/072805** |

### A. CLASSIFICATION OF SUBJECT MATTER

A61C19/04(2006.01)i; G06T17/00(2006.01)i; A61C7/00(2006.01)i

According to International Patent Classification (IPC) or to both national classification and IPC

### B. FIELDS SEARCHED

Minimum documentation searched (classification system followed by classification symbols)

IPC: A61C, G06T, G16H, A61B,

Documentation searched other than minimum documentation to the extent that such documents are included in the fields searched

Electronic data base consulted during the international search (name of data base and, where practicable, search terms used)

CNTXT, ENTXTC, VEN, CJFD: 牙齿, 矫治, 矫正, 正畸, 整牙, 修整, 治疗, 数字模型, 数字图像, 模型, 图像, 三维, 3D, 数字, 口扫, 口腔扫描, 配准, 匹配, 参考点, 特征点, 参考点集, 点对, 标志点, 权重, 加权, 置信度, 置信, 偏差估计, 偏差分析, 偏差, 评估, 估计, 达成率, dental, orthodontics, digital model, digital, oral scan, regist+, reference point, weight+, confidence, deviation, reference point set, point pair, achievement rate

### C. DOCUMENTS CONSIDERED TO BE RELEVANT

| Category* | Citation of document, with indication, where appropriate, of the relevant passages | Relevant to claim No. |
|---|---|---|
| Y | CN 112992342 A (SHENZHEN TOOLINK TECHNOLOGY CO., LTD.) 18 June 2021 (2021-06-18)<br>description, paragraphs 67-89 and 99-104, and figures 2 and 4 | 1-35 |
| Y | CN 109259875 A (NATIONAL INSTITUTE CORPORATION OF ADDITIVE MANUFACTURING, XI'AN) 25 January 2019 (2019-01-25)<br>description, paragraphs 5-29, and figure 1 | 1-35 |
| Y | CN 113140322 A (SHANGHAI KUOHONG INFORMATION TECHNOLOGY CO., LTD.) 20 July 2021 (2021-07-20)<br>description, paragraphs 33-73 | 34-35 |
| Y | US 2010151404 A1 (ALIGN TECHNOLOGY, INC.) 17 June 2010 (2010-06-17)<br>claims 1-29 | 1-35 |
| Y | CN 114549509 A (SHINING 3D TECH. CO., LTD.) 27 May 2022 (2022-05-27)<br>claims 1-10 | 1-35 |

☑ Further documents are listed in the continuation of Box C.　　☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **10 March 2024** | **25 March 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2024/072805** |

| C. | DOCUMENTS CONSIDERED TO BE RELEVANT | |
| --- | --- | --- |

| Category* | Citation of document, with indication, where appropriate, of the relevant passages | Relevant to claim No. |
| --- | --- | --- |
| A | CN 107260351 A (ANGELALIGN TECHNOLOGY INC.) 20 October 2017 (2017-10-20)<br>entire document | 1-35 |
| A | CN 112807108 A (TSINGHUA UNIVERSITY) 18 May 2021 (2021-05-18)<br>entire document | 1-35 |
| A | CN 115349967 A (BEIJING STOMATOLOGICAL HOSPITAL, CAPITAL MEDICAL<br>UNIVERSITY) 18 November 2022 (2022-11-18)<br>entire document | 1-35 |
| A | CN 110811653 A (SOUTHEAST UNIVERSITY) 21 February 2020 (2020-02-21)<br>entire document | 1-35 |
| A | US 8075306 B2 (ALIGN TECHNOLOGY, INC.) 13 December 2011 (2011-12-13)<br>entire document | 1-35 |

Form PCT/ISA/210 (second sheet) (July 2022)

EP 4 652 964 A1

| INTERNATIONAL SEARCH REPORT | | | | | | International application No. |
|---|---|---|---|---|---|---|
| Information on patent family members | | | | | | PCT/CN2024/072805 |

| Patent document cited in search report | | | Publication date (day/month/year) | Patent family member(s) | | | Publication date (day/month/year) |
|---|---|---|---|---|---|---|---|
| CN | 112992342 | A | 18 June 2021 | None | | | |
| CN | 109259875 | A | 25 January 2019 | None | | | |
| CN | 113140322 | A | 20 July 2021 | None | | | |
| US | 2010151404 | A1 | 17 June 2010 | US | 8591225 | B2 | 26 November 2013 |
| | | | | US | 2014087324 | A1 | 27 March 2014 |
| | | | | US | 9168113 | B2 | 27 October 2015 |
| | | | | US | 2019307531 | A1 | 10 October 2019 |
| | | | | US | 2016008096 | A1 | 14 January 2016 |
| | | | | US | 10368960 | B2 | 06 August 2019 |
| CN | 114549509 | A | 27 May 2022 | None | | | |
| CN | 107260351 | A | 20 October 2017 | None | | | |
| CN | 112807108 | A | 18 May 2021 | None | | | |
| CN | 115349967 | A | 18 November 2022 | None | | | |
| CN | 110811653 | A | 21 February 2020 | None | | | |
| US | 8075306 | B2 | 13 December 2011 | EP | 2155101 | A2 | 24 February 2010 |
| | | | | US | 2014193765 | A1 | 10 July 2014 |
| | | | | US | 9364297 | B2 | 14 June 2016 |
| | | | | US | 2008305451 | A1 | 11 December 2008 |
| | | | | US | 2023157789 | A1 | 25 May 2023 |
| | | | | US | 11819377 | B2 | 21 November 2023 |
| | | | | US | 2012225401 | A1 | 06 September 2012 |
| | | | | US | 8636510 | B2 | 28 January 2014 |
| | | | | WO | 2008149222 | A2 | 11 December 2008 |
| | | | | WO | 2008149222 | A3 | 22 May 2009 |
| | | | | US | 2020237477 | A1 | 30 July 2020 |
| | | | | US | 2016074138 | A1 | 17 March 2016 |
| | | | | US | 10624716 | B2 | 21 April 2020 |

Form PCT/ISA/210 (patent family annex) (July 2022)